# EUROPEAN PATENT SPECIFICATION

(11) **EP 0 349 640 B1**
(45) Date of publication and mention of the grant of the patent: **13.07.1994**
(21) Application number: 88902208.3
(22) Date of filing: 25.02.1988
(51) Int. Cl.: A61M 25/00

(54) **CATHETER EQUIPPED WITH EXPANSIBLE MEMBER AND PRODUCTION THEREOF**
KATHETER MIT AUSDEHNBAREN ELEMENTEN UND HERSTELLUNGSVERFAHREN DAFÜR
CATHETER MUNI D'UN ELEMENT EXPANSIBLE ET PROCEDE POUR SA FABRICATION

(30) Priority: 27.02.1987 JP 46195/87; 03.03.1987 JP 47977/87; 05.11.1987 JP 279698/87; 05.11.1987 JP 279699/87; 15.01.1988 JP 7231/88
(43) Date of publication of application: 10.01.1990
(73) Proprietor: TERUMO KABUSHIKI KAISHA, Tokyo 151 (JP)
(72) Inventor: NOBUYOSHI, Masakiyo, Kitakyusyu-shi Fukuoka 803 (JP); SUGIYAMA, Yoshiaki, Fuji-shi Shizuoka 417 (JP); SAGAE, Kyuuta, Fuji-shi Shizuoka 417 (JP)
(74) Representative: Casalonga, Axel
(86) International application number: JP8800202
(87) International publication number: WO8806465

(56) References cited:
- EP-A- 0 102 422
- FR-A- 2 380 786
- JP-A- 5 838 565
- JP-Y- 5 321 910

## Description

The present invention relates to a catheter equipped with an expansible member used for the therapy of a stricture portion inside a blood vessel, as well as a production method thereof. In particular, the present invention relates to a catheter equipped with an expansible member for expanding a stricture portion inside a blood vessel thereby improving the blood flow on the periphery side of the stricture portion, as well as a production method thereof.

Heretofore, as a catheter equipped with an expansible member for expanding a stricture portion inside a blood vessel, there has been so-called Gruentich type disclosed, for example, in U.S. Patent Specification No. 4195637. Further, there has been used a so-called Simpson-Robert type as disclosed, for example, in U.S. Patent Specification No. 4323071.

Previously, adaptable cases for vasodilatation have been localized stricture near coronary artery from an anatomical point of view and it has been restricted, for example, to lesion of about 15 - 20 mm length, monobranched lesion, not-calcified lesion, etc. Then, for enlarging the range of the adaptable cases further, a catheter equipped with an expansible member of a low profile shape having the same structure but narrowed only at the tip has been considered in addition to the standard type of the above-mentioned type, which can be adaptable to the stricture in the peripheral blood vessel or to the severer stricture (sub-complete clogging).

The so-called Gruentich type catheter as described above is formed with a catheter tube having two lumens and an expansible member attached near the tip thereof. Then, one of the lumens opens at the tip of the catheter to constitute a channel for a guide wire and a tip pressure measurement. The other of the lumens is in communication with the inside of the expansible member on the base end thereof, to constitute a flow channel for a vasographic contrast media etc. under pressure, there expanding the expansible member. Then, the catheter is formed with flexible synthetic resin.

Further, the so-called Simpson-Robert type catheter has a coaxial double walled structure comprising an inner tube having a first lumen whose tip is open and an outer tube allowing the inner tube to be inserted to the inside thereof and, further, having an expansible member being attached to the tip thereof, in which a second lumen is formed between the inner surface of the outer tube and the outer surface of the inner tube. Then, an ultrafine metal pipe is disposed in the second lumen for removing bubbles. Also in the catheter of this type, like that the so-called Gruentich type, the catheter for the vasodilation of the blood vessel is made of a flexible synthetic resin.

As has been described above, the catheter is made of the flexible synthetic resin so that it can be inserted safely to the inside of the blood vessel. Since it is made of flexible synthetic resin, it can be inserted into the blood vessel and, moreover, gives less damages to the blood vessel walls. On the other hand, the flexibility may cause flexion of the catheter during insertion into the blood vessel. Furthermore, for displacing and rotating the tip of the catheter in a delicate manner, the catheter is moved forward-to-backward or rotated in a delicate fashion at the proximal portion, thereby transmitting the torque to the tip. Further, for inserting the tip and, further, the expansible member of the catheter into the stricture portion of the blood vessel, the operation for enforcing the catheter is applied at the proximal portion of the catheter. However, it is a drawback that the torque and the enforcing force are absorbed by the flexibility of the catheter and, thus, are less transmitted as far as the tip, therefore worsening the fine operability.

FR-A-2 380 786 discloses a catheter equipped with an expansible member comprising an inner tube having a first lumen whose tip is opened, an outer tube disposed coaxially with said inner tube, having the tip thereof at a position recessed by a predetermined length from the tip of said inner tube and forming a second lumen, an expansible member having its tip portion fitted to said inner tube and its proximal portion to said outer tube, and communicating with said second lumen near said proximal portion, a first opening communicating with said first lumen disposed at the proximal portion of said inner tube, and a second opening communicating with said second lumen disposed at the proximal portion of said outer tube.

In view of the above, the object of the present invention is to provide a catheter equipped with an expansible member with no risk of flexion during insertion to the inside of the blood vessel, having high transmission efficiency of the torque and the enforcing force given to the proximal portion of the catheter and of excellent operability.

Further, a conventional double-walled tube type catheter equipped with an expansible member, has been produced by forming the outer tube and the expansible member integrally and securing the tip of the expansible member on the extension of the outer tube to the tip of the inner tube.

Further, a so-called double-lumen catheter having a second lumen in the wall of a tubular member forming the catheter has been produced by inserting an extension tube from the tip to the inside of either one of the lumens of the tubular member, securing the tip of an expansible member to the tip of the extending tube, and securing the proximal portion of the expansible member to the tip of the double lumen catheter.

In addition, for producing a catheter equipped with an expansible member for dilatating a stricture portion in an endotract, it has been known to use a method of producing a catheter equipped with an expansible member referred to a Simpson-Robert type, for example, as disclosed in U.S. Patent Specification No. 4411055. According to the description of said U.S. patent specification, an expansible member is disposed integrally to the tip of an outer tube forming a second lumen for fluid for expanding the expansible member between it and an inner tube whose tip is open for forming a first lumen, in which the expansible member is formed in the course of the production steps by closing the tip of the outer tube, heating the vicinity on the proximal portion of the closed portion and applying pressure from the proximal portion.

However, in the former method, since the expansible member is formed integrally with the outer tube, it has been difficult to provide the catheter and the expansible member with physical properties required respectively therefor. In addition, there has been a great possibility that the length and the thickness of the expansible member are not sufficiently made uniform and the reproducibility for the outer diameter of the expansible member upon expansion has not completely been satisfactory. Further, the later method requires a step of fitting and securing an extending tube whose tip is open to the tip of one of the double lumens, which makes the operation complicate.

Further, in the conventional production method, it has also been difficult to form an expansible member of uniform wall thickness.

In view of the above, it is an object of the present invention to provide a method of producing a catheter equipped with an expansible member capable of optionally setting the length and the wall thickness of the expansible member, as well as easily producing a catheter equipped with an expansible member.

A further object of the present invention is to provide a method of producing an expansible member used for a catheter equipped with an expansible member, by which the length and the wall thickness of the expansible member can be made uniform and which can produce an expansible member with high reproducibility for the outer diameter of the expansible member upon inflation.

A catheter equipped with an expansible member according to the present invention comprises an inner tube having a first lumen whose tip is open, an outer tube disposed coaxially with the inner tube, having the tip thereof at a position recessed by a predetermined length from the tip of the inner tube and forming a second lumen between it and the outer surface of the inner tube, a contractible or foldable expansible member having a tip portion and a proximal portion, the proximal portion being fitted to the outer tube and the tip portion being fitted to the inner tube, and communicating with the second lumen near the proximal portion, a first opening communicating with the first lumen disposed at the proximal portion of the inner tube and a second opening communicating with the second lumen disposed at the proximal portion of the outer tube, and a rigidity imparting member disposed in at least one of the inner and outer tubes so as to extend in an axial direction.

A method of production of a catheter equipped with an expansible member according to the present invention comprises a step of forming an inner tube having a lumen which opens from the tip to the rear end, a step of forming an outer tube having a lumen which opens from the tip to the rear end and having an inner diameter larger than the outer diameter of the inner tube and being shorter by a predetermined length than the inner tube, a step of disposing a rigidity imparting member at at least one of said inner and outer tubes, a step of separatly forming a contractible or foldable expansible member having a tip portion and a proximal portion, a step of inserting the inner tube into the outer tube, a step of securing the proximal portion of the expansible member to the tip of the outer tube and a step of securing the tip of the expansible member to the tip of the inner tube.

The method of producing an expansible member used for a catheter equipped with an expansible member according to the present invention comprises a step of forming a thermoplastic resin tube and then heating an expansible member forming portion of the tube, a step of disposing the heated expansible member forming portion of the tube in an expansible member molding die the inner surface of which is formed in a shape obtainable when the expansible member is inflated, a step of bringing the heated expansible member forming portion of the tube disposed in the expansible member molding die into close contact with the inner surface of the molding die by pressurizing the inside of the tube, a step of cooling the expansible member forming portion of the tube, a step of removing the expansible member molding die from the tube, and a step of cutting the molded expansible member portion of the tube.

The present invention is now explained by reference to several non limitative examples illustrated in the enclosed drawings where :
Fig. 1 is an enlarged cross sectional view for the tip portion of one embodiment of a catheter equipped with an expansible member according to the present invention.
Fig. 2 is a view illustrating the proximal portion in one embodiment of a catheter equipped with an expansible member according to the present invention.
Fig. 3 is a cross sectional view for the inner tube of the catheter equipped with the expansible member shown in Fig. 1.
Fig. 4 is a cross section view of the catheter equipped with the expansible member taken along line I-I in Fig. 1.
Fig. 5 is a cross sectional view of the catheter equipped with an expansible member taken along line II-II in Fig. 1.
Fig. 6 is an enlarged cross sectional view for the tip portion of another embodiment of a catheter equipped with an expansible member according to the present invention.
Fig. 7 is a cross sectional view of the catheter equipped with the expansible member taken along line III-III in Fig. 6.
Fig. 8 is a cross sectional view of the catheter equipped with the expansible member taken along line IV-IV in Fig. 6.
Fig. 9 is a cross sectional view of the catheter equipped with the expansible member taken along line V-V in Fig. 6.
Fig. 10 is an enlarged cross sectional view for the tip portion of another embodiment of a catheter equipped with an expansible member according to the present invention.
Fig. 11 is a cross sectional view of the catheter equipped with the expansible member taken along line VI-VI in Fig. 10.
Fig. 12 is a cross sectional view of the catheter equipped with the expansible member taken along line VII-VII in Fig. 10.
Fig. 13 is a cross sectional view of the catheter equipped with the expansible member taken along line VIII-VIII in Fig. 10.
Fig. 14 is an explanatory view for the method of applying a rigidity imparting member over the inner tube in the method of producing a catheter equipped with an expansible member in accordance with the present invention.
Fig. 15 is a cross sectional view taken along line IX-IX in Fig. 14.
Figs. 16, 17, 18 and 19 are explanatory views for the production steps of an expansible member in the method of producing a catheter equipped with an expansible member according to the present invention.
Fig. 20 is an enlarged cross sectional view for one embodiment of an expansible member produced by the method of producing the expansible member according to the present invention.
Fig. 21 is a cross sectional view of an expansible member molding die for use in the method of producing the expansible member according to the present invention.
Fig. 22 is an enlarged cross sectional view of a thermoplastic resin tube used for the method of producing the expansible member according to the present invention.
Figs. 23, 24, 25, 26 and 27 are explanatory views for illustrating the steps for the method of producing an expansible member according to the present invention.
Fig. 28 is an explanatory view for the step of securing an outer tube hub to the outer tube in the method of producing a catheter equipped with an expansible member according to the present invention.
Fig. 29 is an explanatory view for the step of securing an inner tube hub to the inner tube in the method of producing a catheter equipped with an expansible member according to the present invention.
Fig. 30 is an explanatory view for the step of securing an expansible member to the outer tube in the method of producing a catheter equipped with an expansible member according to the present invention.
Fig. 31 is an explanatory view for the step of securing an expansible member to the inner tube in the method of producing a catheter equipped with an expansible member according to the present invention.
Figs. 32 and 33 are explanatory views for the step of securing the inner tube hub and the outer tube hub in the method of producing a catheter equipped with an expansible member according to the present invention.
Figs. 34, 35, 36, 37 and 38 are respectively explanatory views for illustrating the function of the catheter equipped with the expansible member according to the present invention.

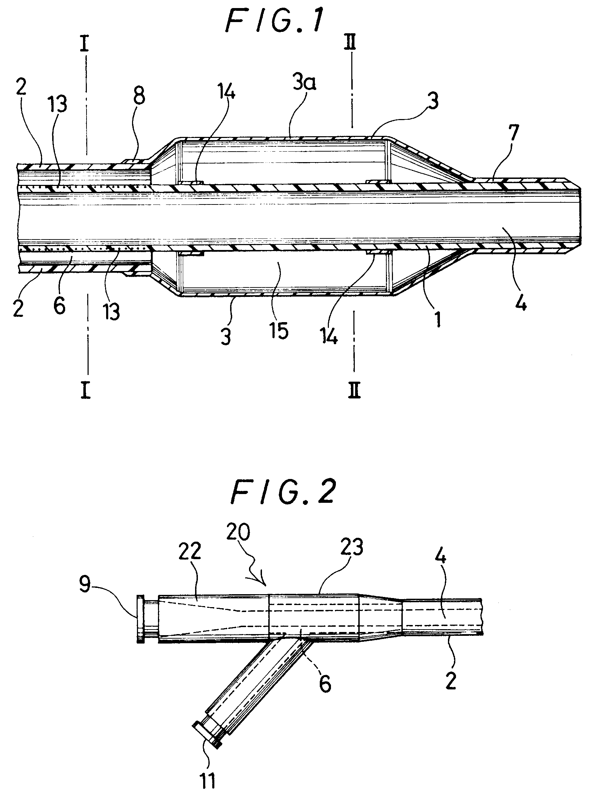

The catheter equipped with an expansible member according to the present invention, as shown in Figs. 1 and 2, comprises an inner tube 1 having a first lumen 4 whose tip is opened, an outer tube 2 disposed coaxially with the inner tube 1, having the tip thereof at a position recessed by a predetermined length from the tip of the inner tube 1 and forming a second lumen 6 between it and the outer surface of the inner tube 1, a contractible or foldable expansible member 3 having a tip portion 7 and a proximal portion 8, the proximal portion 8 being fitted to the outer tube 2 while the tip portion 7 being fitted to the inner tube 1, and communicating with the second lumen 6 near the proximal portion, a first opening 9 disposed at the proximal portion of the inner tube 1 and communicating with the first lumen 4 and a second opening 11 disposed at the proximal portion of the outer tube 2 and communicating with the second lumen 6, and a rigidity imparting member 13 disposed in at least one of the inner tube 1 and the outer tube 2 so as to extend in an axial direction.

The catheter equipped with an expansible member according to the present invention is formed with a catheter main body comprising the inner tube 1, the outer tube 2 and the expansible member 3, and a branched hub 20.

The inner tube 1 has the first lumen 4 opened at the tip. The first lumen 4 is a lumen for inserting a guide wire therethrough and in communication with the first opening 9 forming a guide wire port disposed to the branched hub 20 described later. The inner tube 1 has an outer diameter of 0.40 to 2.50 mm, preferably, 0.55 to 2.40 mm and an inner diameter of 0.25 to 2.35 mm, preferably, 0.30 to 1.80 mm.

Then, the diameter of the tip portion of the inner tube 1 is preferably reduced in a tapered shape toward the tip, because it can facilitate the insertion of the catheter into the blood vessel.

The material for forming the inner tube 1 preferably has a certain extent of flexibility and, for example, there can be used thermoplastic resin such as polyolefin such as polyethylene, polypropylene, ethylene-propylene copolymer and ethylene-vinyl acetate copolymer, polyvinyl chloride, polyamide elastomer, polyester and polyurethane, silicone rubber, latex rubber, etc., the thermoplastic resin being preferred and polyolefin being particularly preferred.

Further, the rigidity imparting member 13 is disposed at at least one of the inner tube 1 and the outer tube 2. In the embodiment shown in Fig. 1, the rigidity imparting member 13 is disposed at the inner tube 1. The rigidity imparting member 13 serves to prevent the flexion of the catheter main body at the bent portion and, further, improve the torque transmission efficiency and the enforcing force of the catheter main body. By disposing the rigidity imparting member 13, flexion of the catheter main body at the bent portion can be prevented. Further, when the catheter main body is rotated at the proximal portion of the catheter main body, the rotation can surely be transmitted as far as the tip portion, the operability can be improved and introduction of the tip portion of the catheter into the highly strictured portion in the blood vessel is facilitated. Further, when the enforcing operation is applied to the catheter main body at the proximal portion of the catheter main body, the enforcing force can surely be transmitted to the tip portion, making it easy to insert the tip portion and the expansible member portion of the catheter into the stricture portion in the blood vessel.

Preferably, the rigidity imparting member 13 is disposed at least from the proximal end of the inner tube 1 as far as the vicinity at the tip portion of the outer tube 2. Further, the rigidity imparting member may also be disposed over the entire length of the inner tube 1. Further, for preventing the end portion of the rigidity imparting member from protruding beyond the tip of the catheter, the rigidity imparting member may not be disposed to the tip portion.

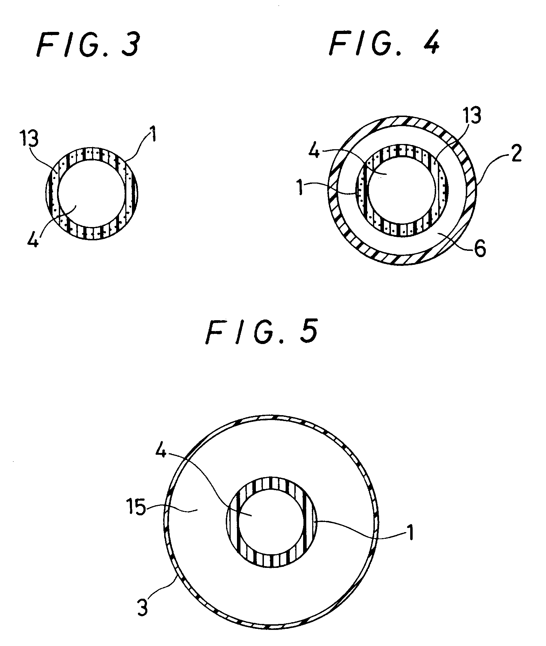

As the rigidity imparting member 13, a mesh-like rigidity imparting member is preferred. The mesh-like rigidity imparting member is preferably, a braided member formed with wire material, particularly, metal wire. As the metal wire, those metal wires, for example, made of stainless steels, elastic metals, super elastic alloys, shape memory alloys, etc. with the wire diameter of 0.01 to 0.2 mm, preferably, 0.03 to 0.1 mm may suitably be used. Then, the mesh-like rigidity imparting member can be formed by winding the metal wire as described above around the Outer surface of the inner tube 1 in the mesh-like manner. Further, as shown in Fig. 3 representing the cross section of the inner tube 1, it is desirable that the rigidity imparting member disposed at the outer surface of the inner tube 1 is embedded in the outer surface of the inner tube 1 so as to make the outer surface smooth. This method can be applied by forming the inner tube 1 with a thermoplastic resin, winding the rigidity imparting member around the outer surface thereof, then, heating the inner tube 1 from the outside (for example, by inserting the inner tube through a heating dice) and embedding the rigidity imparting member in the outer surface of the inner tube. Further, the rigidity imparting member may also be formed by winding synthetic fibers such as polyamide, polyester, and polypropylene fibers as the wire material around the outer surface of the inner tube 1.

The outer tube 2 allows the inner tube 1 to be inserted therethrough and is disposed at such a position where the tip thereof is at a position recessed by a predetermined length from the tip of the inner tube. As shown in Fig. 4, which is a cross sectional view of the catheter equipped with the expansible member taken along line I-I in Fig. 1, a second lumen 6 is formed with the inner surface of the outer tube 2 and the outer surface of the inner tube 1. Thus, the second lumen constitutes a lumen having a sufficient volume. Then, the second lumen 6 is in communication at the tip thereof with the rear end at the inside of the expansible member 3. Further, the second lumen 6 is in communication at the rear end thereof with a second opening 11 of the branched hub 20 forming an injection port for injecting a fluid for inflating the expansible member (for example, vasographic contrast liquid).

As the material for forming the outer tube 2, those having a certain extent of flexibility are preferred and, for example, there can be used thermoplastic resin such as polyolefin such as polyethylene, polypropylene, ethylene-propylene copolymer and ethylene-vinyl acetate copolymer, polyvinyl chloride, polyamide elastomer, polyester and polyurethane, silicone rubber, latex rubber, etc., the thermoplastic resin being preferred and polyolefin being particularly preferred.

Further, the rigidity imparting member may be disposed at the outer tube 2 instead of disposing the rigidity imparting member at the inner tube 1. By disposing the rigidity imparting member, flexion of the catheter main body at the bent portion can be prevented. Further, this can also increase the torque transmission efficiency of the catheter main body and, when the catheter main body is rotated at the proximal portion of the catheter main body, the rotation can surely be transmitted to the tip portion, the operability can be improved, making it easy to introduce the tip portion, thus, the expanding member of the catheter to the highly stricture portion in the blood vessel. As the rigidity imparting member, those explained for the inner tube 1 can be used suitably. The outer tube 2 has an outer diameter of 0.75 to 4.30 mm, preferably, 1.00 to 4.00 mm and an inner diameter of 0.70 to 3.80 mm, preferably, 0.80 to 3.00 mm. Further, the difference between the outer diameter of the inner tube 1 and the inner diameter of the outer tube 2 is 0.30 to 3.40 mm, preferably, 0.50 to 1.20 mm.

In another embodiment, the rigidity imparting member may be disposed at least at either one of the inner tube 1 and the outer tube 2, and it may be disposed at both of the inner tube and the outer tube.

The expansible member 3 is contractible or foldable and it is contracted or folded at the outer circumference of the inner tube 1 in a state not inflated. Then, the expansible member 3 has such a portion at least partially made to a substantially cylindrical shape for enabling to inflate the stricture portion in the blood vessel, and the embodiment shown in Fig. 1 has a substantially cylindrical portion 3a having approximately equal diameter. The substantially cylindrical portion described above may or may not be a completely circular cylinder but it may be a polygonal cylindrical shape. Then, the proximal portion 8 of the expansible member 3 is secured in a liquid-tight manner to the tip portion of the outer tube 2 by means of adhesives or fusion. Further, the tip portion 7 is secured to the tip portion of the inner tube 1 also in a liquid-tight manner.

Then, as shown in Fig. 5 representing the cross sectional view of the catheter equipped with an expansible member taken along line II-II in Fig. 1, the expansible member 3 forms an inflating space 15 between the inner surface thereof and the outer surface of the inner tube 1. The inflating space 15 is in communication along the entire circumference at the proximal portion thereof with the second lumen 6. Thus, since the second lumen having a relatively large volume is in communication with the proximal end of the expansible member 3, injection of expanding fluid in the expansible member 3 from the second lumen is facilitated.

As the material for forming the expansible member 3, those having a certain extent of flexibility are preferred and there can be used, for example, thermoplastic resin such as polyolefin such as polyethylene, polypropylene, ethylene-propylene copolymer, ethylene-vinyl acetate copolymer and cross-linked ethylene-vinyl acetate copolymer, polyvinyl chloride, polyamide elastomer, polyester and polyurethane, silicone rubber, latex rubber, etc., the thermoplastic resin being preferred and the cross-linked ethylene-vinyl acetate copolymer being particularly preferred.

Further, the forward and the backward portions of the cylindrical portion 3a of the expansible member 3 extended to the secured portions 7 and 8 with the inner tube 1 and the outer tube 2 are tapered.

As the size of the expansible member 3, the cylindrical portion, when inflated, has an outer diameter of 1.50 to 35.00 mm, preferably, 2.00 to 30.00 mm and a length of 10.00 to 80.00 mm, preferably, 15.00 to 75.00 mm. The entire length of the expansible member 3 is 15.00 to 120.00 mm, preferably, 20.00 to 100.00 mm.

Further, as shown in Fig. 1, it is preferred that markers 14 made of X-ray impermeable material (for example, made of gold, platinum or alloy thereof) are disposed to the outer surface of the inner tube 1, at a position nearer to the proximal end than the secured portion of expansible member 3 with the inner tube 1 and at a position nearer to the tip than the secured portion of the expansible member 3 with the outer tube 2, i.e., at the positions corresponding to both ends of the cylindrical portion 3a of the expansible member 3. This enables to easily confirm the position for the expansible member under X-ray inspection. As the form of the marker 14, a ring made of metal as described above may be attached by calking to the outer surface of the inner tube 1.

Further, in the catheter equipped with an expansible member according to the present invention, it is preferred for facilitating the insertion into the blood vessel and, further, into the guide catheter described later to apply hydrophilic treatment to a portion which is possibly brought in to contact with blood during use, that is, to the outer surface of the outer tube 2 and to the outer surface of the expansible member 3, so that the surfaces show lubricancy. As the hydrophilic treatment, there can be mentioned a method of coating a hydrophilic polymer, for example, poly(2-hydroxyethylmethacrylate), polyhydroxyethylacrylate, hydroxypropylcellulose, methyl vinyl ether - maleic anhydride copolymer, polyethylene glycol, polyacrylamide and polyvinyl pyrrolidone.

As shown in Fig. 2, the branched hub 20 comprises an inner tube hub 22 and an outer tube hub 23. The inner tube hub 22 has a first opening 9 for forming a guide wire port and it is secured to the proximal portion of the inner tube 1. Further, the outer tube hub 23 has a second opening 11 for forming of an injection port, which is secured to the proximal portion of the outer tube 2. The inner tube hub 22 is secured to the outer tube hub 23 so as to seal the proximal portion of the outer tube hub 23.

As the material for forming the branched hub, there can be suitably used a thermoplastic resin such as polycarbonate, polyamide, polysulfone, polyarylate, methacrylate - butylene - styrene copolymer, etc. Further, instead of providing the branched hub, each of the first lumen and the second lumen may be attached, for example, with a tube having a port member disposed at the proximal end for forming an opening in a liquid seal manner.

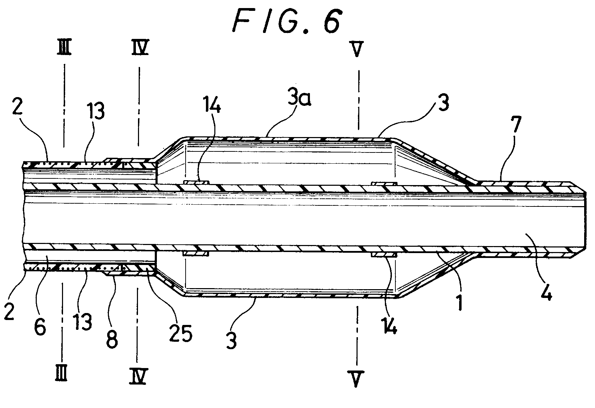

Explanation will now be made for another embodiment of a catheter equipped with an expansible member according to the present invention shown in Fig. 6.

The catheter equipped with an expansible member of the embodiment shown in Fig. 6 comprises an inner tube 1 having a first lumen 4 whose tip is open, an outer tube 2 disposed coaxially with the inner tube 1, having the tip thereof at a position recessed by a predetermined length from the tip of the inner tube 1 and forming a second lumen 6 between it and the outer surface of the inner tube 1, a contractible or foldable expansible member 3 having a tip portion 7 and a proximal portion 8 in which the proximal portion 8 is fitted to the outer tube 2 and the tip portion 7 is fitted to the inner tube 1, and communicating with the second lumen 6 near the proximal portion, a first opening communicating with the first lumen 4 disposed at the proximal portion of the inner tube 1 and a second opening 11 communicating with the second lumen 6 disposed at the proximal portion of the outer tube 2, the outer tube 2 having a rigidity imparting member extending in an axial direction and a portion not disposed with the rigidity imparting member at the tip thereof.

The catheter equipped with an expansible member of this embodiment is to be explained referring to the drawing mainly regarding the differences between it and the catheter equipped with the expansible member shown in Fig. 1.

The catheter equipped with the expansible member according to the present invention shown in Fig. 6 comprises a catheter main body having an inner tube 1, an outer tube 2 and an expansible member 3, a rigidity imparting member 13 formed in the outer tube 2 and an annular member 25 forming a portion not provided with the rigidity imparting member at the tip of the outer tube 2 having the rigidity imparting member, and a branched hub 20.

The inner tube 1 has a first lumen 4 whose tip is open. The first lumen 4 is a lumen for inserting a guide wire therethrough and communicating with a first opening 9 disposed to the branched hub 20 for forming the guide wire port shown in Fig. 2.

Then, the diameter at the tip of the inner tube 1 is preferably reduced in a tapered shape toward the side of the tip, since this facilitates the insertion of the catheter to the stricture portion inside the blood vessel. For the material and the size for forming the inner tube 1, those described above can be preferably used.

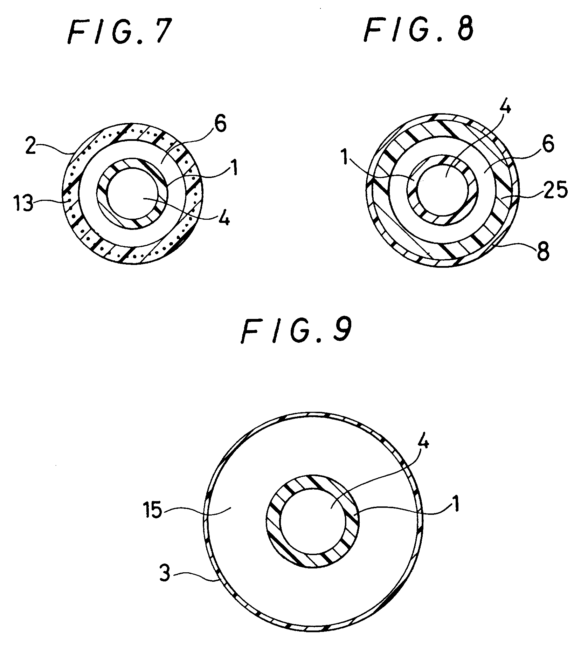

The outer tube 2 allows the inner tube 1 to be inserted therethrough and has a tip disposed at a position recessed somewhat from the tip of the inner tube. As shown in Fig. 7 which is a cross sectional view of the catheter equipped with the expansible member taken along line III-III in Fig. 6, the second lumen 6 is defined with the inner surface of the outer tube 2 and the outer surface of the inner tube 1. Thus, the second lumen 6 constitutes a lumen having a sufficient volume. Then, the second lumen 6 is in communication at the tip thereof with the rear end inside of the expansible member 3, while the proximal of the second lumen 6 is in communication with the second opening 11 of the branched hub 20 for forming an injection port for injecting a fluid (for example, vasographic contrast liquid) for inflating the expansible member. For the material and the size for forming the outer tube 2, those described previously are preferably used.

Further, the outer tube 2 is provided with the rigidity imparting member 13. As the rigidity imparting member 13, those as described previously can be used preferably.

Further, an annular member 25 is attached at the tip of the outer tube 2 having the rigidity imparting member 13, for forming a portion not provided with the rigidity imparting member 13. The annular member 25 is secured to the cut face at the tip of the outer tube 2 by means of fusion using heat, supersonic wave, high frequency wave, etc., bonding by using adhesive, solvent, etc. The annular member 25 serves to prevent the rigidity imparting member 13 from protruding externally out of the tip face of the outer tube 2 and also prevent the damage of the expansible member 3 due to the protruded portion of the rigidity imparting member. Fig. 7 is a cross sectional view of the catheter equipped with the expansible member taken along line III-III in Fig. 6, showing that the rigidity imparting member 13 is disposed at the outer tube 2. Further, Fig. 8 is a cross sectional view of the catheter equipped with the expansible member taken along line IV-IV in Fig. 6, showing that the annular member 25 disposed at the tip of the outer tube 2 is not provided with the rigidity imparting member. Accordingly, even if the inner surface of the expansible member 3 should be in contact with the tip of the outer tube 2 when the expansible member 3 described later is contracted or folded, since the member is in contact with the portion of the annular member 25 that is not provided with the rigidity imparting member, there is no worry that the expansible member 3 is damaged or destructed.

The annular member 25 may have any length so long as it has a length capable of covering the rigidity imparting member 13 protruding from the tip of the outer tube 2. However, if it is too long, since the portion has no rigidity imparting member and the torque transmission efficient is reduced, it is, preferably, less than about 10 mm and, more preferably, about 2 to 7 mm. As the material for forming the annular member 25, it is preferred that the material is identical or similar with that for the outer tube to be connected therewith. It is further preferred that the material is somewhat flexible and there can be used, for example, thermoplastic resin such as polyolefin such as polyethylene, polypropylene, ethylene-propylene copolymer and ethylene-vinyl acetate copolymer, polyvinyl chloride, polyamide elastomer, polyester and polyurethane, silicone rubber, latex rubber, etc., the thermoplastic resin being preferred and polyolefin being more preferred.

The method of forming a portion not provided with the rigidity imparting member 13 at the tip of the outer tube 2 having the rigidity imparting member 13 may be conducted by a method other than using the annular member described above. For instance, for forming the rigidity imparting member at the outer surface from a position somewhat distant from the tip of the outer tube to the rear end of the outer tube formed with the thermoplastic resin, metal wires such as made of stainless steel, elastic metal, superelastic alloy, shape memory alloy, etc. may be wound as wire material in a mesh-like manner and, further, the outer tube 2 wound around with the metal wire may be heated from the outside (for example, by inserting the outer tube through the heating dice), so that the rigidity imparting member is embedded in the outer wall of the outer tube 2, thereby forming a portion not having the rigidity imparting member at the tip. Further, a resin which is adhesive to the material for forming the outer tube 2 may be coated to the tip of the outer tube 2 at such a thickness that the rigidity imparting member situated at the tip does not protrude to the outside, thereby forming a portion having no rigidity imparting member.

The expansible member 3 is contractible or foldable, and, in a state not-inflated, it is contracted or folded to the outer circumference of the inner tube 1. Then, the expansible member 3 at least has a portion of substantially cylindrical shape for easily dilatating the stricture portion of the blood vessel and the embodiment shown in Fig. 6 has substantially cylindrical portion 3a of about equal diameter. The substantially cylindrical portion may not always be a completely circular cylinder but it may be a polygonal cylinder. Then, the expansible member 3 is secured at the proximal portion 8 to the tip of the outer tube 2 by means of adhesive or heat fusion in a liquid-tight manner, while the tip 7 thereof is secured in the same manner as that for the tip of the inner tube 1 in a liquid-tight manner. As shown in Fig. 9 which is a cross sectional view of the catheter equipped with the expansible member taken along line V-V in Fig. 6, the expansible member 3 forms an inflating space 15 between the inner surface of the expansible member 3 and the outer surface of the inner tube 1. The inflating space 15 is in communication with the second lumen 6 at the entire circumference of the rear end portion. Thus, since the second lumen having a large volume is in communication with the rear end of the expansible member 3, expanding fluid can easily be injected from the second lumen to the inside of the expansible member 3. As the material and the size for forming the expansible member 3, those described above can be used suitably. Further, the portions of the expansible member 3 from the front and rear of the cylindrical portion 3a to the portions 7 and 8 secured with the inner tube 1 and outer tube 2 are tapered.

It is preferred that markers 14 made of X-ray impermeable material (for example, gold, platinum or alloys thereof) are disposed to the outer surface of the inner tube 1, at the position nearer to the rear end from the securing portion between the expansible member 3 and the inner tube 1, and at the position nearer to the tip from the secured portion between the expansible member 3 and the inner tube 2, that is, at the both ends of the cylindrical portion 3a of the expansible member 3.

In the catheter equipped with the expansible member according to the present invention, it is preferred to apply hydrophilic treatment to those portions possibly being brought into contact with blood upon use, that is, to the outer surface of the outer tube 2 and the outer surface of the expansible member 3 for facilitating the insertion into the blood vessel and, further, into the guide catheter described later, so that they exhibit lubricancy when brought into contact with blood, etc. As for the hydrophilic treatment, those described previously can be used suitably.

The branched hub 20 is identical with that explained referring to Fig. 2.

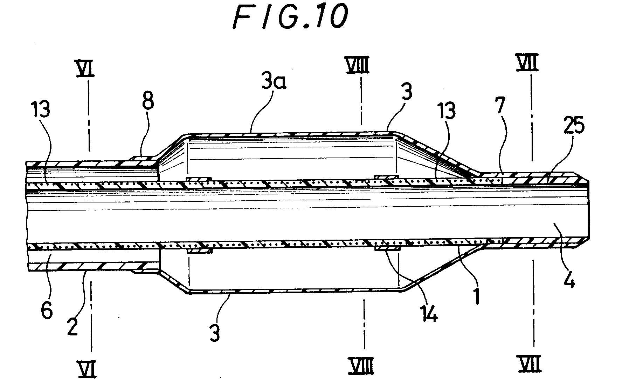

Explanation will now be made of another embodiment of a catheter equipped with an expansible member according to the present invention shown in Fig. 10.

The catheter equipped with an expansible member of the embodiment shown in Fig. 10 comprises an inner tube 1 having a first lumen 4 whose tip is open, an outer tube 2 disposed coaxially with the inner tube 1, having the tip thereof at a position recessed by a predetermined length from the tip of the inner tube 1 and forming a second lumen 6 between it and the outer surface of the inner tube 1, a contractible or foldable expansible member 3 having a tip portion 7 and a proximal portion 8 in which the proximal portion 8 is fitted to the outer tube 2 and the tip portion 7 is fitted to the inner tube 1, and communicating with the second lumen 6 near the proximal portion, a first opening 9 communicating with the first lumen 4 disposed at the proximal portion of the inner tube 1, a second opening 11 communicating with the second lumen 6 disposed at the proximal portion of the outer tube 2, the inner tube 1 having a rigidity imparting member extending from the proximal portion to the tip in an axial direction and a portion not disposed with the rigidity imparting member at the tip thereof.

The embodiment of the catheter equipped with the expansible member according to the present invention shown in Fig. 10 comprises a catheter main body having an inner tube 1, an outer tube 2 and an expansible member 3, a rigidity imparting member 13 formed in the inner tube 1 and an annular member 25 forming a portion not provided with the rigidity imparting member at the tip of the inner tube 1 having the rigidity imparting member, and a branched hub 20.

The inner tube 1 has a first lumen 4 whose tip is open. The first lumen 4 is a lumen for inserting a guide wire therethrough and communicating with a first opening 9 disposed to the branched hub 20 for forming the guide wire port shown in Fig. 2.

The diameter at the tip of the inner tube 1 is preferably reduced in a tapered shape toward the side of the tip, since this facilitates the insertion of the catheter to the stricture portion inside the blood vessel. For the material and the size for forming the inner tube 1, those described above can be preferably used.

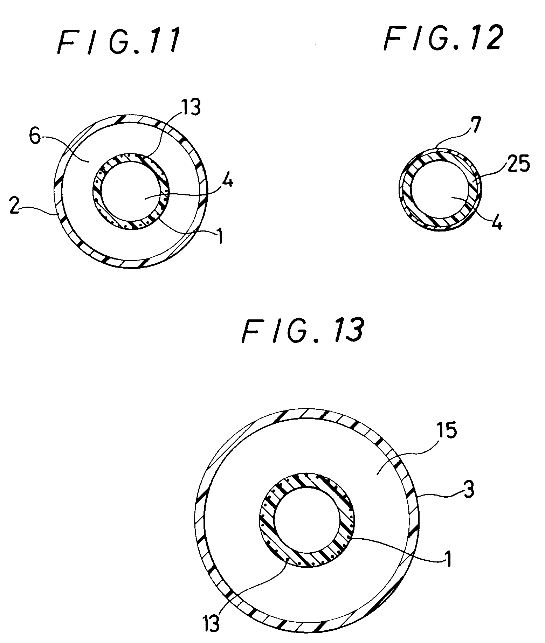

The inner tube 1 is provided with the rigidity imparting member 13. Further, an annular member 25 is attached at the tip of the inner tube 1 having the rigidity imparting member 13, for forming a portion not provided with the rigidity imparting member 13. As the rigidity imparting member 13, those as described previously can be used preferably. The annular member 25 is secured to the cut face at the tip of the inner tube 1 by means of fusion using heat, supersonic wave, high frequency wave, etc., bonding by using adhesive, solvent, etc. for serving to prevent the rigidity imparting member 13 from protruding externally out of the tip face of the inner tube 1. Fig. 11 is a cross sectional view of the catheter equipped with the expansible member taken along line VI-VI in Fig. 10, showing that the rigidity imparting member 13 is disposed at the inner tube 1. Fig. 12 is a cross sectional view of the catheter equipped with the expansible member taken along line VII-VII in Fig. 10, showing that the annular member 25 disposed at the tip of the inner tube 1 is not provided with the rigidity imparting member. The annular member 25 may have any length so long as it has a length capable of covering the rigidity imparting member 13 protruding from the tip of the inner tube 1. However, if it is too long, since the portion has no rigidity imparting member and the torque transmission efficient is reduced, it is, preferably, less than about 10 mm and, more preferably, about 2 to 7 mm. As the material for forming the annular member 25, it is preferred that the material is identical or similar with that for the inner tube to be connected therewith. It is further preferred that the material is somewhat flexible and there can be used, for example, thermoplastic resin such as polyolefin such as polyethylene, polypropylene, ethylene - propylene copolymer and ethylene - vinyl acetate copolymer, polyvinyl chloride, polyamide elastomer, polyester and polyurethane, silicone rubber, latex rubber, etc., the thermoplastic resin being preferred and polyolefin being more preferred.

Thus, even if the rigidity imparting member 13 is disposed at the inner tube 1, since the tip portion thereof forms a portion not having the rigidity imparting member, the rigidity imparting member does not protrude from the end face at the tip of the inner tube when advancing in a blood vessel thereby preventing the damage to the inner wall of the blood vessel. Then, it is preferred that the diameter of the annular member 25 as the tip of the inner tube 1 forming the portion not having the rigidity imparting member 13 is reduced in a tapered shape toward the tip, since this facilitates the insertion of the catheter into the blood vessel. Further, it is preferred that the rigidity imparting member 13 disposed at the inner tube 1 is extended to a portion corresponding to the contractible or foldable portion of the expansible member 3 (portion excluding the proximal portion secured to the outer tube 2 and the tip portion secured to the inner tube 1) from the proximal portion of the inner tube 1. Such disposition can prevent the inner tube 1 from flexing in the portion of the expansible member thereby enabling to prevent the destruction of the expansible member caused by reflexing, as well as transmit the torque and enforcing force reliably to the tip of the inner tube 1.

The method of forming a portion not provided with the rigidity imparting member 13 at the tip of the inner tube 1 having the rigidity imparting member 13 may be conducted by a method other than using the annular member described above. For instance, for forming the rigidity imparting member at the outer surface from a position somewhat distant from the tip of the inner tube to the rear end of the inner tube formed with the thermoplastic resin, metal wires such as made of stainless steel, elastic metal, superelastic alloy, shape memory alloy, etc. may be wound as wire material in a mesh-like manner and, further, the inner tube wound around with the metal wire may be heated from the outside (for example, by inserting the outer tube through the heating dice), so that the rigidity imparting member is embedded in the outer wall of the inner tube, thereby forming a portion not having the rigidity imparting member at the tip. Further, a resin which is adhesive to the material for forming the inner tube may be coated to the tip of the inner tube 1 at such a thickness that the rigidity imparting member situated at the tip of the inner tube does not protrude to the outside, thereby forming a portion having no rigidity imparting member.

The outer tube 2 allows the inner tube 1 to be inserted therethrough and is disposed at such a position where the tip thereof is at a position recessed by a predetermined length from the tip of the inner tube. As shown in Fig. 11, which is a cross sectional view taken along line VI-VI in Fig. 10, a second lumen 6 is formed with the inner surface of the outer tube 2 and the outer surface of the inner tube 1. Then, the second lumen 6 is in communication at the tip thereof with the rear end at the inside of the expansible member 3, and the second lumen 6 is in communication at the rear end thereof with a second opening 11 of the branched hub 20 forming an injection port for injecting a fluid for inflating the expansible member (for example, vasographic contrast liquid).

As the material for forming the outer tube 2, those described above can suitably be used.

The expansible member 3 is contractible or foldable, and, in a state not-inflated, it is contracted or folded to the outer circumference of the inner tube 1. Then, the expansible member 3 at least has a portion of substantially cylindrical shape for easily dilatating the stricture portion of the blood vessel and the embodiment shown in Fig. 10 has substantially cylindrical portion 3a of about equal diameter. The substantially cylindrical portion may not always be a completely circular cylinder but it may be a polygonal cylinder. Then, the expansible member 3 is secured at the proximal portion 8 to the tip of the outer tube 2 by means of adhesive or heat fusion in a liquid-tight manner. The tip portion 7 is secured in the same manner to the tip of the inner tube 1 in a liquid-tight manner. As shown in Fig. 13 which is a cross sectional view of the catheter equipped with the expansible member taken along line VIII-VIII in Fig. 10, the expansible member 3 forms an inflating space 15 between the inner surface of the expansible member 3 and the outer surface of the inner tube 1. The inflating space 15 is in communication with the second lumen 6 at the entire circumference of the proximal portion. Thus, since the second lumen having a large volume is in communication with the rear end of the expansible member 3, expanding fluid can easily be injected from the second lumen to the inside of the expansible member 3. As the material and the size for forming the expansible member 3, those described above can be used suitably. Further, the portions of the expansible member 3 from the front and rear of the cylindrical portion 3a to the portions 7 and 8 secured with the inner tube 1 and outer tube 2 are tapered.

It is preferred that markers 14 made of X-ray impermeable material (for example, gold, platinum or alloys thereof) are disposed to the outer surface of the inner tube 1, at the position nearer to the rear end from the securing portion between the expansible member 3 and the inner tube 1, and at the position nearer to the tip from the secured portion between the expansible member 3 and the outer tube 2, that is, at the both ends of the cylindrical portion 3a of the expansible member 3.

In the catheter equipped with the expansible member according to the present invention, it is preferred to apply hydrophilic treatment to those portions possibly being brought into contact with blood upon use, that is, to the outer surface of the outer tube 2 and the outer surface of the expansible member 3 for facilitating the insertion into the blood vessel and, further, into the guide catheter described later, so that they exhibit lubricancy when brought into contact with blood, etc. As for the hydrophilic treatment, those described previously can be used suitably.

The branched hub 20 is identical with that explained referring to Fig. 2.

The method of producing a catheter equipped with an expansible member according to the present invention will now be explained referring to the drawings.

The method of producing the catheter equipped with the expansible member according to the present invention comprises a step of forming an inner tube having a lumen opened from the tip to the rear end, a step of forming an outer tube having a lumen opened from the tip to the rear end, with the inner diameter being larger than the outer diameter of the inner tube and shorter by a predetermined length than the inner tube, a step of forming a contractible or foldable expansible member having a tip portion and a proximal portion, a step of inserting the inner tube to the inside of the outer tube, a step of securing the proximal portion of the expansible member to the tip portion of the outer tube and a step of securing the tip portion of the expansible member to the tip portion of the inner tube.

Each of the steps will now be explained referring to the catheter equipped with the expansible member shown in Fig. 1.

The step of forming inner tube 1 having the first lumen 4 communicating from the tip to the rear end can be conducted using flexible material, for example, thermoplastic resin such as polyolefin such as polyethylene, polypropylene, ethylene - propylene copolymer and ethylene - vinyl acetate copolymer, polyvinyl chloride, polyamide elastomer, polyester and polyurethane, silicone rubber or latex rubber by means of extrusion molding followed by cutting to a predetermined length, or injection molding, dipping, etc.

The inner tube 1 has a length of 300 to 2100 mm, preferably, 400 to 1350 mm, an outer diameter of 0.40 to 2.50 mm, preferably, from 0.55 to 2.40 mm and inner diameter of 0.25 to 2.35 mm, preferably, 0.30 to 1.80 mm.

It is preferred to provide the thus formed inner tube 1 with a rigidity imparting member 13 for preventing the flection of the catheter main body at the bent portion and, further, for improving the torque transmission efficiency of the catheter main body. The rigidity imparting member can be formed easily by a method, for example, of applying wire material to the outer surface of the inner tube 1 in a mesh-like manner.

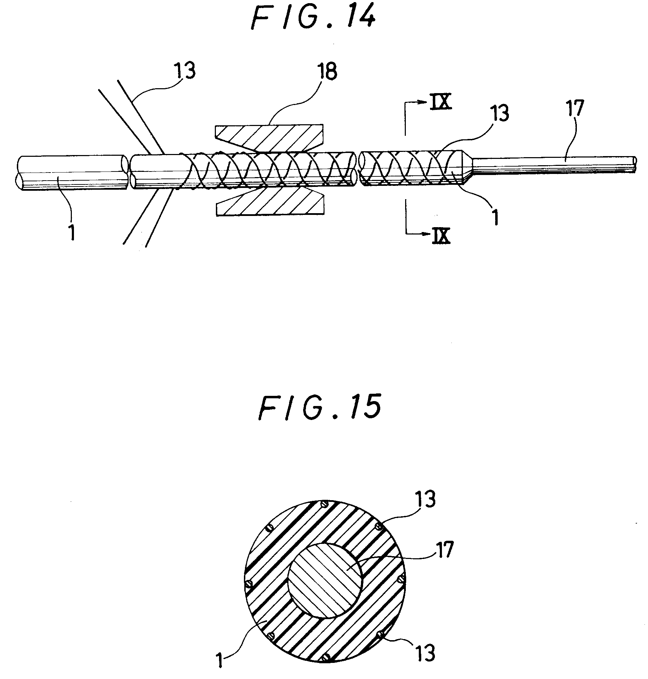

As the wire material, metal wire is preferred and, for example, stainless steel, elastic metal, superelastic alloy or shape memory alloy with a wire diameter of 0.01 to 0.2 mm, preferably, 0.03 to 0.1 mm is preferred. Further, it is preferred that the rigidity imparting member 13 disposed at the outer surface of the inner tube 1 is embedded in the outer surface of the inner tube 1 so as to make the outer surface of the inner tube 1 smooth. This method can be applied, for example, by inserting a metal core 17 through the inner tube 1 formed with a thermoplastic resin and then inserting the inner tube 1 into a heating dice 18 while winding the rigidity imparting member 13 over the inner tube 1 as shown in Fig. 14. Then, in the portion passed through the inside of the heating dice 18, the rigidity imparting member 13 is embedded in the outer surface of the inner tube 1 as shown in Fig. 15 which is a cross sectional view taken along line IX-IX in Fig. 14. Although the inner tube 1 is caused to pass through the heating dice while winding the rigidity imparting member therearound in the previous explanation, the method is not restricted only thereto but the rigidity imparting member may previously be applied over the outer surface of the inner tube which is then inserted through the heating dice.

The rigidity imparting member 13 may be formed by winding, as the wire material, synthetic fibers such as polyamide fibers, polyester fibers or polypropylene fibers around the outer surface of the inner tube 1.

It is preferred to apply coating of a thermoplastic resin to the outer surface of the inner tube 1 in which the rigidity imparting member is embedded and, as the thermoplastic resin, there can be suitably used thermoplastic resin such as polyolefin such as polyethylene, polypropylene, ethylene - propylene copolymer and ethylene - vinyl acetate copolymer, polyvinyl chloride, polyurethane and polyester. It is more preferable to use those materials having high adhesiveness to the outer surface of the inner tube 1, for example, the material identical with or similar to that used for forming the inner tube 1. The thermoplastic resin can be coated easily by a method of inserting the inner tube 1, in which the rigidity imparting member is deposited and embedded, through a die that discharges a coating thermoplastic resin in a molten state.

It is preferred that the diameter at the tip portion of the inner tube 1 is reduced toward the tip in a tapered shape, because this can facilitate the insertion of the catheter into a blood vessel. The fabrication to the tip of the inner tube may be applied after attaching an expansible member 3 described later.

The step of forming the outer tube 2 having the second lumen 6 communicating the tip to the rear end can be applied to using the same flexible material as that for the inner tube 1, for example, thermoplastic resin such as polyolefin such as polyethylene, polypropylene, ethylene - propylene copolymer and ethylene - vinyl acetate copolymer, polyvinyl chloride, polyamide elastomer, polyester and polyurethane, silicone rubber or latex rubber by means of extrusion molding followed by cutting to a predetermined length, injection molding or dipping.

The outer tube 2 has a length of 200 to 2000 mm, preferably, 250 to 1450 mm, an outer diameter of 0.75 to 4.30 mm, preferably, 1.00 to 4.00 mm, and inner diameter of 0.70 to 3.80 mm, preferably, 0.80 to 3.00 mm. The difference between the outer diameter of the inner tube 1 and the inner diameter of the outer tube 2 is from 0.30 to 3.40 mm, preferably, 0.50 to 1.20 mm.

Instead of disposing the rigidity imparting member at the inner tube 1, the rigidity imparting member may be disposed at the outer tube. For forming the rigidity imparting member, the method as explained for the step of forming the inner tube 1 can suitably be utilized. In particular, in a case of disposing the rigidity imparting member at the outer tube, since the outer tube is liable to be in contact with the inner surface of a blood vessel and, in order to reduce the onset of thrombus, it is preferred that the rigidity imparting member disposed at the outer surface of the outer tube is embedded in the outer surface of the outer tube to make the outer surface smooth. For doing this, the method as explained for the step of forming the inner tube 1 can also suitably be used. For making the outer surface of the outer tube more smooth, it is preferred to apply a coating of a thermoplastic resin to the outer surface of the outer tube 2 embedded with the rigidity imparting member. As the thermoplastic resin and the method of coating the same, those explained for the inner tube 1 can suitably be used. The steps of forming the inner tube and the outer tube may be conducted in any order or conducted simultaneously.

Explanation will now be made for the step of forming the contractible or foldable expansible member having the tip portion and the proximal portion.

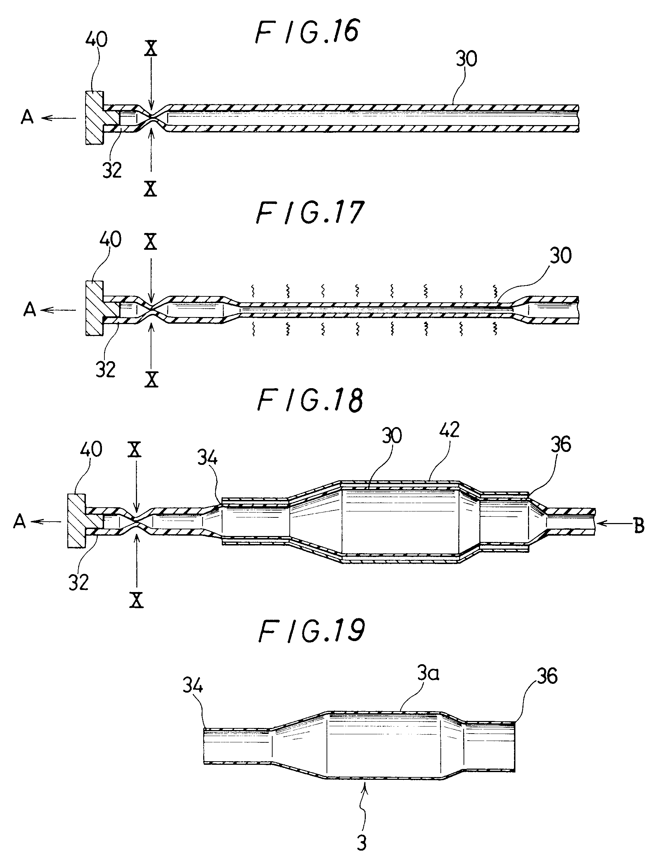

The expansible member 3 is contractible or foldable and, in a state not-inflated, it can be contracted or folded to the outer circumference of the inner tube 1. Then, the expansible member 3 has a substantially cylindrical portion 3a at least a portion of which is substantially cylindrical for easily dilatating the stricture portion of a blood vessel, as shown in Fig. 19. The substantially cylindrical portion may not always be a completely circular cylinder but it may be a polygonal cylinder.

The expansible member 3 preferably has flexibility and can be formed, for example, by using thermoplastic resin such as polyolefin such as polyethylene, polypropylene, ethylene - propylene copolymer, ethylene - vinyl acetate copolymer and cross-linked ethylene - vinyl acetate copolymer, polyvinyl chloride and polyurethane, more preferably, cross-linked ethylene - vinyl acetate copolymer, for example, as shown in Fig. 16 through Fig. 19.

At first, as shown in Fig. 16, a tube 30 made of a thermoplastic resin is formed for forming the expansible member 3 and a tube holder 40 is attached to the end 32 of the tube 30. Further, the lumen of the tube 30 is closed at the portion X-X near the tube holder 40. The method of closing is applied by melting under heating or high frequency wave sealing or by using forceps. The tube 30 closed at the portion X-X is stretched in the direction A while applying a load to the tube holder 40, thereby removing the slackening of the tube 30. Fig. 16 shows the state in which the slackening is removed. The tube 30 removed with the slackening is heated at the portion for forming the expansible member 3 by a heating device (not illustrated) to a temperature near the melting point of the material forming the tube 30 as shown in Fig. 17. The tube 30 is maintained at the heated state, a molding die 42 the inner cavity of which is in a shape obtainable when the expansible member is inflated is fitted over the tube 30, a gas is supplied from the direction of arrow B under pressure to bring the tube 30 at the portion heated in the mold 42 into close contact with the inner wall surface of the molding die 42 as shown in Fig. 18. Then, they are left while maintaining the pressurized state till the tube 30 resumes to the normal temperature and, thereafter, the inside pressure of the tube 30 is made negative to shrink the portion as the expansible member and remove the mold 42. Then, the expansible member 3 can be formed as shown in Fig. 19 by cutting the tube 30 at the tip portion 34 and the rear end portion 36 of the tube 30. Further, if the expansible member is provided with heat shrinkability at least for the tip and the proximal portion thereof, the expansible member 3 can easily be attached to the outer tube 2 and the inner tube 1 by utilizing the heat shrinkability. At least the tip and the proximal portion of the expansible member may be provided with heat shrinkability by a method of forming the expansible member with a cross-linked thermoplastic resin or, not only restricted to such a method of using the cross-linked resin, but also by forming the expansible member in its forming step described above at such a temperature as leaving strain in the expansible member, thereby providing the expansible member with the heat shrinkability. Further, it is also possible to provide heat shrinkability by forming the expansible member in which the inner diameter at the tip thereof is somewhat smaller than the outer diameter of the inner tube and, the proximal portion is somewhat smaller than the outer diameter of the outer tube and then expanding the outer diameter of both ends of the expansible member by stretching. Then, the thus formed expansible member 3 has an outer diameter at the cylindrical portion, when it is inflated, of 1.50 to 35.00 mm, preferably, 2.00 to 30.00 mm, a length of 10.00 to 80.00 mm, preferably, 15.00 to 75.00 mm and the entire length of the expansible member 3 of 15.00 to 120.00 mm, preferably, 20.00 to 100.00 mm.

The step for forming the expansible member may be conducted at any stage, and the sequence in relation with the steps of forming the inner tube and the outer tube described above may be optional.

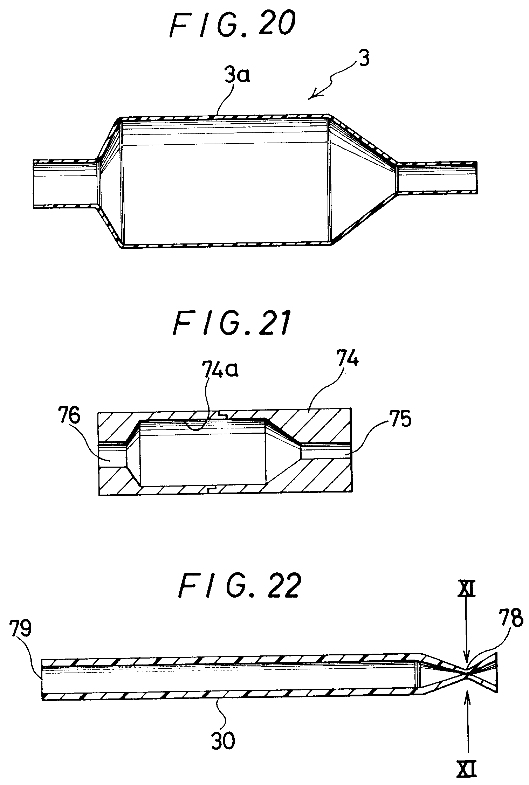

The method of providing the expansible member according to the present invention will now be explained referring to another embodiment in conjunction with Fig. 20 through Fig. 27.

The method of producing the expansible member according to the present invention comprises a step of forming a thermoplastic resin tube and then heating expansible member forming portion of the tube, a step of disposing the heated expansible member forming portion of the tube in an expansible member molding die having the inner surface of which is formed into a shape obtainable when the expansible member is inflated, a step of bringing the heated expansible member forming portion of the tube disposed in the expansible member molding die into close contact with the inner surface of the molding die by pressurizing the inside of the tube, a step of cooling the expansible member forming portion of the tube, a step of removing the expansible member forming die from the tube and a step of cutting the molded expansible member portion off the tube.

Fig. 20 is an enlarged cross sectional view for one embodiment of an expansible member produced in accordance with the present invention. The expansible member 3 is contractible or foldable and, in a state not-inflated, it can be contracted or folded. The expansible member 3 has a tip portion and a proximal portion the diameters of which are reduced respectively each in a tapered shape toward the end and has a substantially cylindrical portion 3a at least a portion of which is substantially cylindrical for easily dilatating the stricture portion of a blood vessel. The substantially cylindrical portion may not always be a complete circular cylinder but it may be a polygonal cylinder.

Fig. 21 shows a cross sectional view of an expansible member forming die 74 used for the method of producing the expansible member according to the present invention. The expansible member molding die 74 is provided with a tip opening 75 and a base end opening 76 and has a cylindrical portion 74a for forming the substantially cylindrical portion 3a of the expansible member 3. Fig. 22 is an enlarged cross sectional view of a tube used for the method of producing the expansible member according to the present invention.

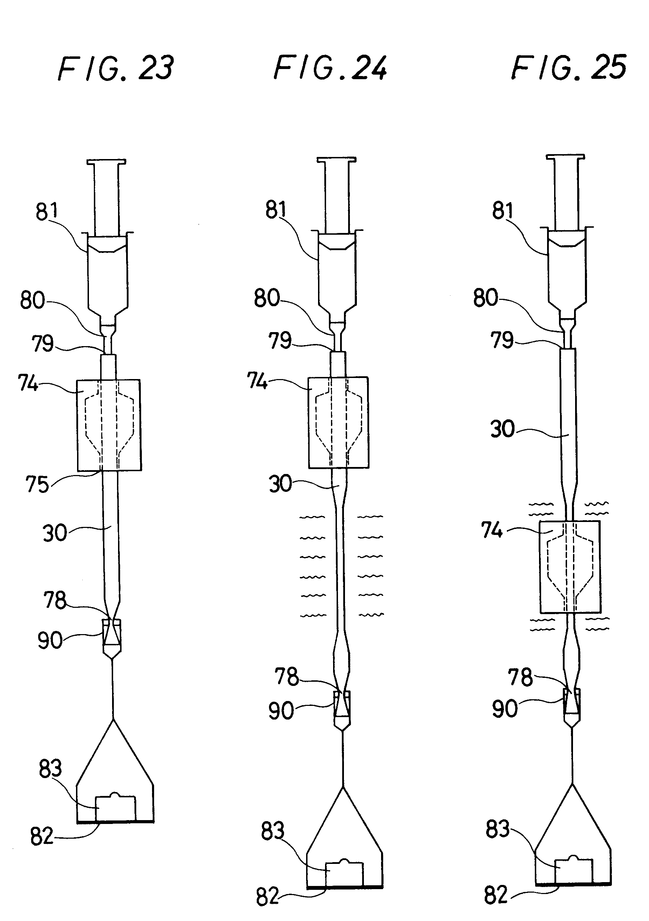

Explanation will now be made for each of the steps for the method of producing the expansible member according to the present invention using explanatory views for the method of producing the expansible member according to the present invention shown in Fig. 23 through Fig. 27.

A thermoplastic resin tube 30 is a tubular body opened at both ends, and the step for forming the tube 30 is conducted by using a material (preferably having flexibility), for example, thermoplastic resin such as polyolefin such as polyethylene, polypropylene, ethylene - propylene copolymer, ethylene - vinyl acetate copolymer and cross-linked ethylene - vinyl acetate copolymer, polyvinyl chloride and polyurethane, preferably, cross-linked thermoplastic resin, particularly preferably, cross-linked ethylene - vinyl acetate copolymer, by means of a known method such as extrusion molding or injection molding. Then, as the cross-linking treatment, although there may be a method of mixing a crosslinker into material for forming the tube, it is preferred to conduct by means of electron-ray irradiation or gamma-ray irradiation without using the crosslinker.

Before the step of heating the expansible member forming portion of the tube 30, one end of the tube 30 is sealed for the pressurizing step subsequently applied to the inside of the tube 30 and a pressurizing means is attached to the other end. Referring specifically, a closed portion 78 is formed by closing at the portion XI-XI at one end of the tube 30 as shown in Fig. 22. The portion XI-XI is closed by binding or heat fusing the tube. Then, as shown in Fig. 23, a pressurizing means 81 such as syringe is attached to the open end 79 of the tube 30. In this case, it is confirmed that air does not leak from the closed portion 78 and the open end 79 which is a connection portion with the pressurizing means 81 even when air is injected under pressure by the pressurizing means 81.

Further, the step of heating the expansible member forming portion of the tube is preferably applied together with a step of stretching the heated expansible member forming portion of the tube 30 in the axial direction of the tube 30. The step of stretching the heated expansible member forming portion of the tube 30 in the axial direction of the tube 30 is preferably conducted by applying a predetermined axial stretching load to the tube 30.

Referring specifically, as shown in Fig. 23, the closed portion 78 of the tube 30 is caught by a chuck 90 having a weight disk 82 attached to the tip. The expansible member molding die 74 is inserted from the tip opening 75 through the opening end 79 of the tube 30 and, subsequently, a pipe-like member such as a needle 80 having a diameter equal with or somewhat larger than the inner diameter of the tube 30 is inserted through the opening end 79, and the pressurizing means 81 such as a syringe is attached to the rear end thereof. The optimum weight of the weight 83 placed on the weight disk 82 is 132 g, for example, in a case of molding a tube made of ethylene - vinyl acetate copolymer and having 1.0 mm outer diameter and 0.45 mm inner diameter as the tube 30 and blow molding an expansible member of 2.5 mm outer diameter by using the tube partially cross-linked by means of electron-rays (geling rate of 90.4 %).

Then, the step of heating the tube 30 is conducted by heating the expansible member forming portion of the tube 30 by means of a known method. Referring specifically, as shown in Fig. 24, the tube 30 is heated by using a heating device such as a heat gun (not illustrated), to heat a resin for forming the tube 30 near the melting point thereof. Then, since the end of the tube 30 is applied with a stretching load in the axial direction, the heated portion is spontaneously stretched in the heating step and, accordingly, the stretching step is applied simultaneously. If an excess load than required is applied as the stretching load on the end of the tube 30, it goes beyond the stretching state and there is a worry that the heated portion of the tube can no more endure the weight and be disconnected. In the case of using the tube 30 with the above-mentioned conditions, it was necessary that the weight of the weight 83 is less than 190 g.

Explanation will now be made for the step of disposing the heated expansible member forming portion of the tube 30 in the expansible member molding die 74 the inner surface of which is in a shape obtainable when the expansible member is inflated and a step of bringing the heated expansible member forming portion of the tube 30 disposed in the expansible member molding die 74 into close contact with the inner surface of the molding die 74.

As the expansible member molding die 74, either one piece die structure or split-die structure may be used, but it is preferred in the case of using the one piece die structure that the expansible member molding die 74 is previously disposed at an optional position of the tube 30 before attaching the pressurizing means 81 to the tube 30, as shown in Fig. 23. This is not necessary in the case of using a split-type molding die.

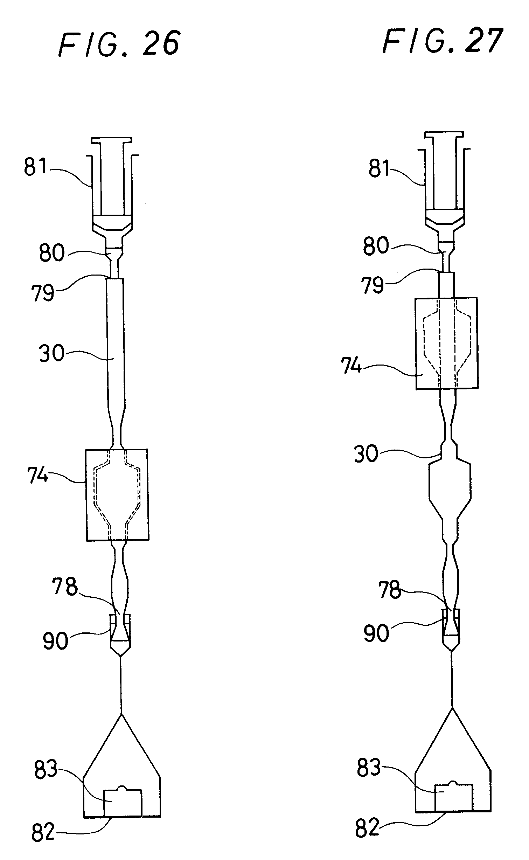

As shown in Fig. 25, the expansible member molding die 74 is set to the heated portion of the tube 30 and, subsequently, as shown in Fig. 26, the inside of the tube 30 is pressurized by the pressurizing means 81 to stretch the tube, by which the thin-walled expansible member forming portion of the tube 30 is brought into close contact with the inner surface of the molding die 74 to conduct inflation molding for the expansible member. Then, by controlling the load applied axially to the tube 30, the wall thickness of the inflation-molded expansible member 3 can be adjusted thereby enabling to produce an expansible member of constant wall thickness. In this case, it is necessary to apply a sufficient pressure by the pressurizing means 81 and blowing the heated portion of the tube 30 to surely bring it into close contact with the inner surface of the expansible member molding die 74. Then, if the stretching load applied to the tube 30 is less than the required weight, it sometimes fails to attain a predetermined thickness for the heated portion of the tube 30 and attain an expansible member of well-defined shape. In the case of using the tube 30 under the above-mentioned conditions, the required minimum load was 50 g.

Explanation will now be made for the step of cooling the expansible member forming portion of the tube 30, the step of removing the expansible member molding die 74 from the tube 30 and the step of cutting the expansible member portion molded to the tube 30.

At first, the step of cooling the expansible member forming portion of the tube 30 can be applied by stopping the heating for the tube 30 and allowing it to cool. Further, it may be conducted by bringing a cooling medium such as air into contact with the molding die 74. Then, the step of removing the expansible member molding die 74 from the expansible member forming portion of the tube 30 is preferably conducted by shrinking the portion of the expansible member 3 formed to the tube 30. The portion of the expansible member 3 can be shrinked by rendering the inside pressure of the tube 30 negative by the pressurizing means 81 attached to the tube 30. Further, in a case of using the split-die structure for the expansible member molding die 74, the molding die 74 may be removed by splitting and it is not always necessary to cause shrinkage to the portion of the expansible member 3. Referring more specifically, as shown in Fig. 26, after inflation molding the expansible member forming portion of the tube 30 under sufficient pressurization, it is cooled as such and, as shown in Fig. 27, the inside pressure in the expansible member and the tube is made negative by using the pressurizing means 81, thereby shrinking the expansible member forming portion and then removing the expansible member molding die from the molded expansible member 3.

If the cooling after blowing is insufficient, the molded expansible member may possibly be shrinked by the heat conducted from the heated portion of the tube 30.

The thus molded expansible member 3 is cut out at the tip portion 3b and the rear end portion 3c of the expansible member 3 from the tube 30, thereby producing the expansible member according to the present invention as shown in Fig. 20.

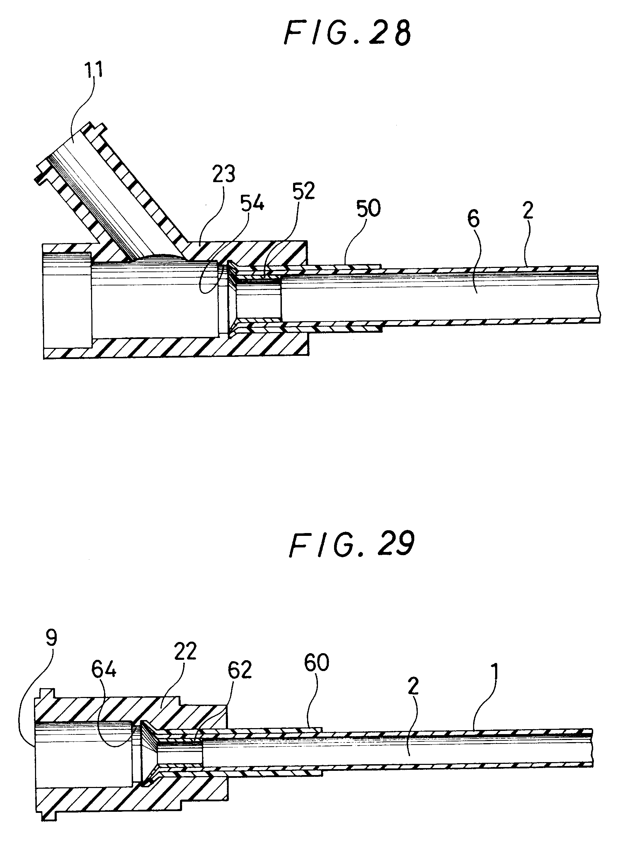

Explanation will now be made for the step of disposing the opening 11 to the proximal end of the outer tube 2 in communication with the lumen 6 of the outer tube. The opening 11 is preferably disposed by attaching the outer tube hub 23 having an opening to the proximal portion of the outer tube 2. Explanation is to be made for such a case as an example referring to Fig. 28.

At first, a flection-preventive tube 50 is attached to the end of the outer tube 2. The attaching can be made by a method of using a heat shrinkable tube 50 for preventing the reflexing, fitting the heat shrinkable tube 50 formed such that the inner diameter after heat shrinkage is somewhat smaller than the outer diameter of the outer tube 2 to the end of the outer tube 2, then causing it to shrink by heating (for example by exposing to hot blow). Then, the outer hub 23 is attached to the inner tube 2 to which the flection-preventive tube 50 is attached. Referring to the attaching method, a stopper pin 52 the outer diameter of which for the portion other than the rear end portion is substantially equal with the inner diameter of the outer tube 2 and having a rear end portion with enlarged diameter is inserted to the rear end of the outer tube 2, the outer tube 2 is inserted from its tip into the outer tube hub 23 and then enforced till the rear end of the stopper pin 52 goes beyond a protrusion 54 disposed to the inner surface of the outer tube hub 23. Further, adhesive may be coated for securing to the contact face between the outer tube hub 23 and the flection-preventive tube 50. As the material forming the outer tube hub, there can be suitably used thermoplastic resin such as polycarbonate, polyamide, polysulfon, polyarylate, methacrylate - butylene - styrene copolymer, etc.

Then, the step of forming the opening 11 in communication with the lumen 6 of the outer tube 2 at the base end portion of the outer tube 2 may be conducted at any stage so long as it is after the formation of the outer tube 2. Preferably, it is conducted after applying a step of securing the proximal portion of the expansible member 3 to the tip portion of the outer tube 2 described later. The sequence with respect to the step of forming the inner tube 1 may be optional.

Explanation will now be made for the step of forming the opening 9 in communication with the lumen 4 of the inner tube 1 at the proximal portion of the inner tube 1. The opening 9 is preferably formed by attaching the inner hub 22 having the first opening 9 to the proximal portion of the outer tube 1. Explanation is to be made for such a case, as an example, referring to Fig. 29.

At first, a flection-preventive tube 52 is attached to the end of the inner tube 1. The attaching can be made by a method of using a heat shrinkable tube for the flection-preventive tube 60 fitting the heat shrinkable tube 60 formed such that the inner diameter after heat shrinkage is somewhat smaller than the outer diameter of the inner tube 1 to the end of the inner tube 1, then causing it to shrink by heating (for example by exposing to hot blow). Then, the inner tube hub 22 is attached to the inner tube 1 to which the flection-preventive tube 60 is attached. Referring to the attaching method, a stopper pin 62 the outer diameter of which for the portion other than the rear end portion is substantially equal with the inner diameter of the inner tube 1 and having a rear end portion with enlarged diameter is inserted to the rear end of the inner tube 1, the inner tube 1 is inserted from its tip into the inner tube hub 22 and then enforced till the rear end of the stopper pin 62 goes beyond a protrusion 64 disposed to the inner surface of the inner tube hub 22. Further, adhesive may be coated for securing to the contact face between the inner tube hub 22 and the flection-preventive tube 60. As the material forming the inner tube hub, there can be suitably used thermoplastic resin such as polycarbonate, polyamide, polysulfon, polyarylate, methacrylate - butylene - styrene copolymer, etc.

Then, the step of forming the opening 9 in communication with the lumen 4 of the inner tube at the proximal portion of the inner tube 1 may be conducted at any stage so long as it is after the formation of the inner tube 1. The sequence for the step of forming the outer tube 2, the step of forming the second opening 11 in communication with the lumen 6 of the outer tube 2 at the proximal portion of the outer tube 2 and a step of forming the expansible member 3 may be optional.

Explanation will now be made for the step of securing the proximal portion 8 of the expansible member 3 to the tip portion of the outer tube 2.

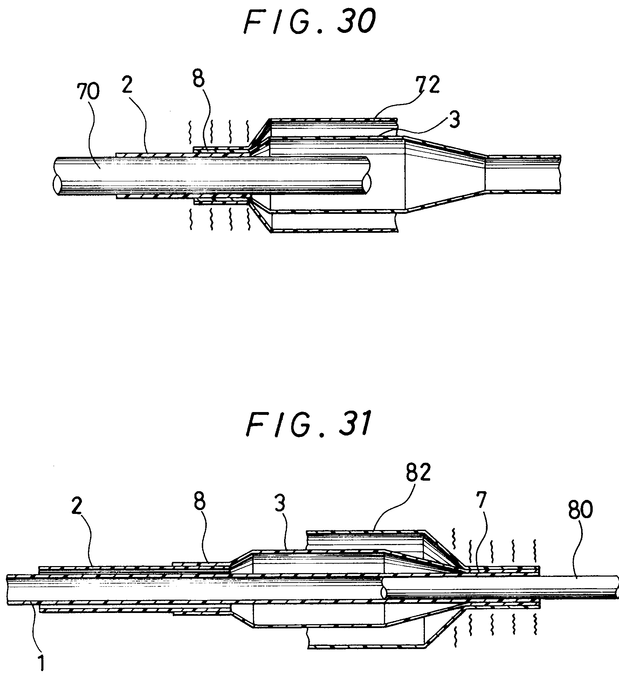

For securing the proximal portion 8 of the expansible member 3 to the tip portion of the outer tube 2, as shown in Fig. 30, a metal core 70 having an outer diameter substantially equal with or somewhat smaller than the inner diameter of the outer tube 2 is inserted from the tip or the rear end of the outer tube 2, and the expansible member 3 is inserted from the tip portion of the metal core 70 such that the tip end of the proximal portion 8 of the expansible member coincides with the tip of the outer tube 2. Then, a glass mold 72 for bonding is fitted so as to situate over the proximal portion 8 of the expansible member 3 and the glass mold 72 is heated by a heating device (not illustrated), thereby securing the proximal portion of the expansible member 3 to the tip portion of the outer tube 2. Further, when the expansible member 3 having a heat shrinkable proximal portion 8 is used, it can easily be secured because of heat shrinkage by the heating of the glass mold 72. After securing the proximal portion 8 of the expansible member 3 to the tip portion of the outer tube 2, and then leaving the glass mold 72 till the normal temperature, the glass mold 72 is retracted from the bonded portion and the metal core 70 is drawn to easily secure the proximal portion 8 of the expansible member 3 with the tip portion of the outer tube 3.

Although a glass mold is used in the foregoing explanation, it is not always restricted thereto and, for example, bonding metal die may be used. Further, a high frequency transmitting electrode may be fitted to the proximal portion of the expansible member 3 by using a metal core 70, and fused by means of high frequency wave to attain securing. Furthermore, it may be fused by using supersonic waves. The step of securing the proximal portion 8 of the expansible member 3 to the tip portion of the outer tube 2 may be conducted at any stage so long as it is after the formation of the outer tube 1 and the expansible member 3. The sequence for the step of forming the inner tube 1 and the step of forming the opening 9 in communication with the lumen 4 of the inner tube at the proximal portion of the inner tube 1 may be optional. Further, in a case of using an axially splittable die as the glass mold or metal mold and, further, in a case of securing by means of high frequency wave or supersonic wave, the sequence with respect to the step of forming the opening 11 in communication with the lumen 6 of the outer tube at the proximal portion of the outer tube 2 may be optional. In order to reduce the possibility of giving damage to the expansible member upon production, it is preferred to apply the above-mentioned step after forming the opening 11 at the proximal portion of the outer tube 2 for communication with the lumen 6 of the outer tube.

Explanation will now be made for securing the inner tube hub 22 having an opening and attached to the proximal portion of the inner tube 1 and the outer tube hub 23 attached to the proximal portion of the outer tube 2.

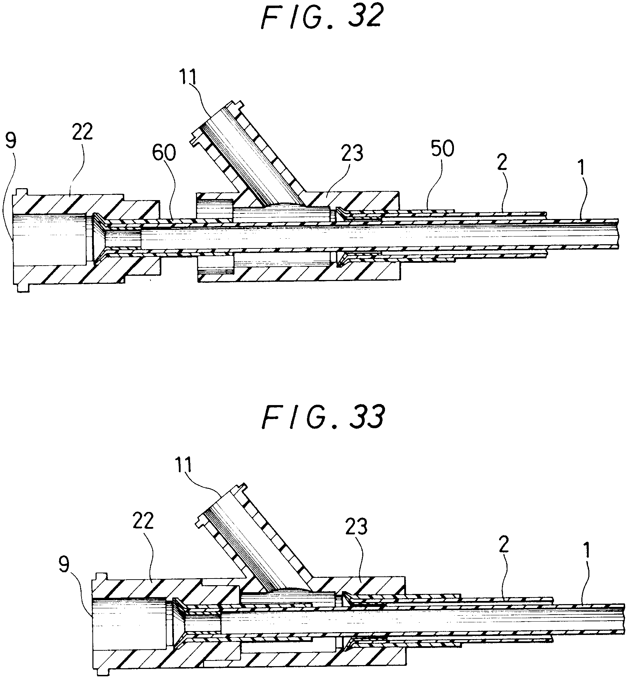

As shown in Fig. 32, the inner tube 1 is inserted from its tip from the rear end of the outer tube hub 23 attached to the proximal portion of the outer tube 2. In this instance, a metal core may be inserted to the inside of the inner tube 1 so as to prevent reflexing of the inner tube 1. Furthermore, as shown in Fig. 33, the tip portion of the inner tube hub 22 is inserted into the rear end of the outer tube hub 23 for bonding. Further, in this case, adhesive may be coated to the bonded portion between the inner tube hub 22 and the outer tube hub 23 to surely secure both of them.

The step of securing the inner tube hub 22 having an opening attached to the proximal portion of the inner tube 1 and the outer tube hub 23 attached to the proximal portion of the outer tube 2 may be applied at any time so long as it is after the application of the step of forming the inner tube 1, a step of disposing the inner tube hub 22 to the proximal portion of the inner tube 1 and, further, a step of forming the outer tube 2 and a step of disposing the outer tube hub 23 to the proximal portion. Preferably, it is applied desirably after forming the expansible member 3 and after the step of securing the expansible member 3 and the outer tube 2.

Explanation will now be made for the step of securing the tip portion 7 of the expansible member 3 to the tip portion of the inner tube 1.

Explanation is to be made for the step of securing the tip portion 7 of the expansible member 3 to the tip portion of the inner tube 1 referring to an example which is conducted after securing the expansible member 3 and the outer tube 1 and securing the inner tube hub 22 having an opening attached to the proximal portion of the inner tube 1 and the outer tube hub 23 attached to the proximal portion of the outer tube 2.

As shown in Fig. 31, a metal core 80 having an outer diameter substantially equal with or somewhat smaller than the inner diameter of the inner tube 1 is inserted to the inside of the inner tube 1 from the tip or the rear end of the inner tube 1. Since the expansible member 3 is secured to the outer tube 2, the inner tube 1 is inserted to the inside of the outer tube 2, and the inner tube hub 22 is secured with the outer tube hub 23, the inner tube 1 is protruded from the tip of the outer tube 2 and, further, from the tip of the expansible member 3. Then, the tip portion of the inner tube 1 protruded from the tip of the expansible member 3 is cut being aligned with the tip of the expansible member 3. Then, a bonding glass mold 82 is fitted from the tip of the metal core 80 so as to situate on the tip portion 7 of the expansible member 3 and the glass mold 82 is heated by a heating device (not illustrated) thereby securing the tip portion 7 of the expansible member 3 to the tip portion of the inner tube 1. Preferably, by using an expansible member 3 whose tip portion 7 is formed so as to be heat shirinkable, it can be secured easily because of heat shrinkage by heating from the glass mold 82. After securing the tip portion 7 of the expansible member 3 to the tip portion of the inner tube 1, and then allowing the glass mold 82 to cool to the normal temperature, glass mold is retracted from the bonded portion and the metal core 80 is withdrawn, by which the tip portion 7 of the expansible member 3 and the tip portion of the inner tube 1 can be secured with ease.

Although a glass mold is used in the foregoing explanation, it is not restricted only thereto but a bonding metal mold may be used for instance. Alternatively, a metal core 80 is used and an electrode for high frequency transmission may be fitted to the tip portion 7 of the expansible member 3 and securing may be conducted by fusing with high frequency wave. Furthermore, fusing may be conducted by using supersonic waves. Further, the step of securing the tip 7 of the expansible member 3 to the tip portion of the inner tube 1 is preferably conducted as the final step after securing the expansible member 3 and the outer tube 1, and after securing the inner tube hub 22 having the first opening and attached to the proximal portion of the inner tube 1 and the outer tube hub 23 attached to the proximal portion of the outer tube 2 since the production becomes easy.

The above-mentioned step may be conducted at any stage so long as it is after the formation of the inner tube 1 and the expansible member 3 in a case where an axially splittable mold is used for the glass mold or metal mold or in a case of securing by means of high frequency or supersonic wave. The sequence for the step of forming the first opening 9 in communication with the lumen 6 of the inner tube at the proximal portion of the inner tube 1, the step of forming the outer tube 2 and the step of forming the second opening 11 in communication with the lumen 6 of the outer tube at the proximal portion of the outer tube 2 may be optional.

Further, it is preferred, after securing the tip portion of the expansible member to the tip portion of the inner tube, to apply tip fabrication so as to reduce the outer diameter at the tip of the inner tube in a tapered shape toward the tip end, or so that the tip constitutes a rounded tip. The tip fabrication can easily be applied by inserting the tip of the inner tube into a mold (for example, glass mold or metal mold) having such an inner shape as conforming the aimed shape of the tip, heating the mold and then heat-deforming the tip of the inner tube along with the shape at the inside of the mold. Further, the tip of the inner tube may be fabricated by using a metal mold as the mold and applying high frequency or supersonic wave for transmission to the mold.

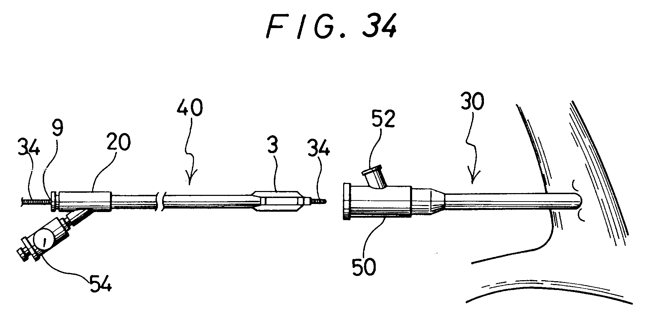

Explanation will now be made for the function of a catheter 40 having an expansible member according to the present invention using the catheter equipped with the expansible member of the embodiment shown in Fig. 1 through Fig. 5 while referring to explanatory views in Fig 34 through Fig. 38.

Before applying dilatating cure to a stricture portion caused in a blood vessel, it is preferred that the air in the catheter equipped with the expansible member is removed as much as possible. In view of the above, suction-injection means such as a syringe is attached to the second opening 11 of the catheter according to the present invention, liquid (vasodiographic contrast liquid, etc.) is charged in the cylinder and suction and injection are repeated, so that the air in the second lumen and the expansible member is removed and replaced with the liquid.

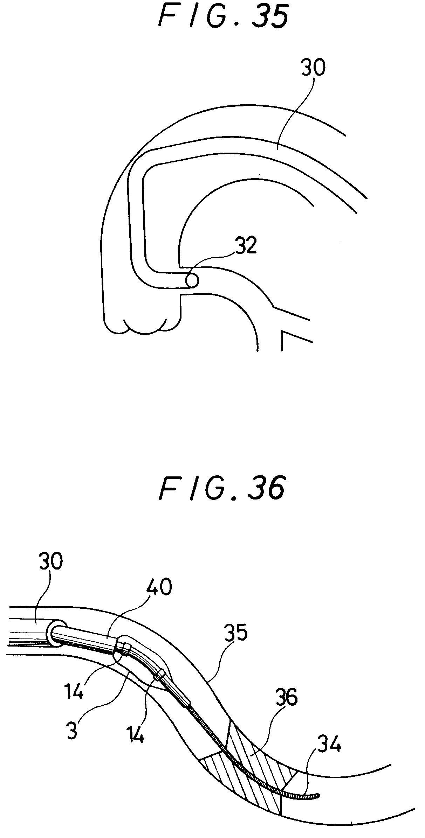

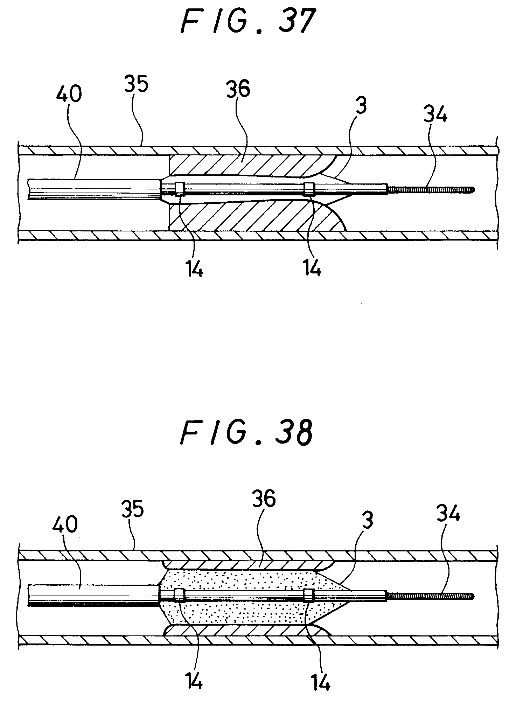

Then, upon inserting the catheter 40 with the expansible member into a human body, a blood vessel is secured in the human body by means of Seldinger method, etc., then a guide wire for guide catheter (not illustrated) is retained in the blood vessel, the guide catheter is inserted therealong into the blood vessel and, as shown in Fig. 35, the guide catheter 30 is retained at the inlet 32 of coronary artery having aimed lesion part. Then, the guide wire for the guide catheter is withdrawn. As shown in Fig. 34, the catheter 40 equipped with the expansible member according to the present invention having the guide wire 34 for the catheter equipped with the expansible member inserted therethrough is inserted by means of the Y-shaped connector 50 disposed at the rear end of the guide catheter 30. Insertion into the blood vessel is conducted in a state where the guide wire 34 for the catheter equipped with the expansible member is protruded by several centimeters from the tip of the catheter 40 equipped with the expansible member. The catheter 40 equipped with the expansible member advances in the guide catheter 30 and, as shown in Fig. 36, leaves the tip of the guide catheter 30 and enters the blood vessel 35 having the aimed lesion part. Subsequently, the guide wire 34 for the catheter equipped with the expansible member is advanced to the aimed lesion part, passed through the stricture portion 36 and then retained. The catheter 40 equipped with the expansible member is advanced in the blood vessel 35 along the guide wire 34. After the catheter 40 equipped with the expansible member has reached a position near the stricture portion 36, the expansible member 3 is situated in the stricture portion 36 under X-ray perspection by using X-ray impermeable markers 14 disposed on the inner tube as the reference marks as shown in Fig. 37. Subsequently, vasodiographic contrast liquid is injected at a pressure from several atm. to ten and several atm. by means of an injector 54 equipped with a pressure gauge connected to the second opening forming the injection port of the catheter 40 equipped with the expansible member as shown in Fig. 34, thereby compressing and dilatating the stricture portion 36 as shown in Fig. 38. Then, the expansible member 3 is caused to shrink and retract from the stricture portion 36 of the dilatated blood vessel. Then, the vasodiographic contrast liquid is injected through the contrast liquid injection port 52 of the Y-shaped connector 50 of the guide catheter 30 shown in Fig. 34 to confirm the state of blood stream at the peripheral side. When improvement of the blood stream at the peripheral side is recognized, the catheter 40 equipped with the expansible member and the guide wire 34 for the catheter equipped with the expansible member are withdrawn and, thereafter, the guide catheter is withdrawn and blood is stopped under pressure to complete the operation.

Since the catheter equipped with the expansible member according to the present invention comprises an inner tube having a first lumen whose tip is open, an outer tube disposed coaxially with the inner tube, having the tip thereof at a position recessed by a predetermined length from the tip of the inner tube and forming a second lumen between it and the outer surface of the inner tube, a contractible or foldable expansible member having a tip portion and a proximal portion in which the proximal portion is fitted to the outer tube and the tip portion is fitted to the inner tube, and communicating with the second lumen near the proximal portion, a first opening communicating with the first lumen disposed at the base end portion of the inner tube, a second opening communicating with the second lumen disposed at the proximal portion of the outer tube, and a rigidity imparting member disposed in at least one of the inner and the outer tubes so as to extend in an axial direction and, particularly, it comprises the rigidity imparting member, there is no worry that the catheter is flexed during insertion into a blood vessel and, further, torque and enforcing force can reliably be transmitted to the tip by displacing, rotating or enforcing the catheter in a delicate manner at the proximal portion of the catheter in a case where the tip of the catheter is displaced or rotated in a delicate manner, thereby providing excellent operability. Further, since the second lumen in communication near the proximal portion of the expansible member and to which the inflating fluid for the expansible member is charged is formed between the inner tube and the outer tube, it has a relatively large volume and, accordingly, the inflating fluid can easily be charged even if it has high flow resistance such as the vasodiographic contrast liquid.

Since the method of producing a catheter equipped with an expansible member according to the present invention comprises a step of forming an inner tube having a lumen opened from the tip to the rear end, a step of forming an outer tube having a lumen opened from the tip to the rear end, having an inner diameter greater than the outer diameter of the inner tube and shorter by a predetermined length than the inner tube, a step of forming a contractible or foldable expansible member having a tip portion and a proximal portion, a step of inserting the inner tube into the outer tube, a step of securing the proximal portion of the expansible member to the tip portion of the outer tube, and a step of securing the tip portion of the expansible member to the tip portion of the inner tube and particularly, since the expansible member is formed separately, the length and the wall thickness of the expansible member can be made optionally. Further, since the inner tube and the outer tube are independent of each other, there is no requirement for providing a complicate step of inserting and securing a narrow extending tube into either one of the lumens with small inner diameter as in the case of producing a catheter of double lumen type equipped with an expansible member, and the catheter equipped with the expansible member can be produced with ease.

Since the method of producing the expansible member used for the catheter equipped with the expansible member, comprises a step of molding a thermoplastic resin tube and then heating the expansible member forming portion of the tube, a step of disposing the heated expansible member forming portion of the tube into an expansible member molding die the inner surface of which is in such a shape as obtainable when the expansible member is inflated, a step of pressurizing the inside of the tube thereby bringing the heated expansible member forming portion of the tube disposed in the expansible member molding die in close contact with the inner surface of the molding die, a step of cooling the expansible member forming portion of the tube, a step of removing the expansible member molding die from the tube and a step of cutting the expansible member portion formed to the tube and, particularly, since inflation molding is applied in the expansible member molding die the inner surface of which is in such a shape as obtainable when the expansible member is inflated, the length and the thickness of the expansible member can be made uniform and, accordingly, an expansible member of high reproducibility for the outer diameter of the expansible member upon inflation can easily be produced.

## Claims

1. A catheter equipped with an expansible member which comprises an inner tube (1) having a first lumen (4) whose tip is open, an outer tube (2) disposed coaxially with said inner tube, forming a second lumen (6) between it and the outer surface of said inner tube (1), a contractible or foldable expansible member (3) having a tip portion fitted to said inner tube (1) and a proximal portion fitted to said outer tube (2), and communicating with said second lumen near said proximal portion, a first opening (9) communicating with said first lumen (4) disposed at the proximal portion of said inner tube (1), a second opening (11) communicating with said second lumen (6) disposed at the proximal portion of said outer tube (2), the tip of said outer tube (2) being at a position recessed by a predetermined length from the tip of said inner tube (1), characterized in that a rigidity imparting member (13) is disposed at at least one of said inner and outer tubes so as to extend in the axial direction.

2. A catheter according to claim 1 wherein said expansible member (3) is made of polyolefin, polyvinylchloride, polyamide elastomer or polyester.

3. A catheter according to claim 1 or 2, wherein said expansible member (3) has a substantially cylindrical portion (3a) having approximately equal diameter.

4. A catheter according to any claim 1 to 3, wherein a forward and a backward portion of said cylindrical portion (3a) of said expansible member are tapered.

5. A catheter according to claim 2, wherein said polyolefin is polyethylene, polypropylene, ethylene-vinyl acetate copolymer or cross-linked ethylene-vinyl acetate copolymer.

6. A catheter according to any preceding claim, wherein said first opening and said second opening are disposed at a branched hub (20) attached to the proximal ends of said inner tube (1) and said outer tube (2).

7. A catheter according to any claims 1 to 6, wherein an outer surface of said outer tube and said expansible member has been submitted to a hydrophilic treatment so as to exhibit lubricancy when said outer surface is brought into contact with blood during use.

8. A catheter according to claim 7, wherein said hydrophilic treatment is carried out by coating a hydrophilic polymer selected from poly(2-hydroxyethylmethacrylate), polyhydroyethylacrylate, hydroxypropylcellulose, methyl vinyl ether - maleic anhydride copolymer, polyethylene glycol, polyacrylamide and polyvinyl pyrrolidone.

9. A catheter according to any preceding claim, wherein said rigidity imparting member (13) is disposed at said inner tube (1).

10. A catheter according to any of claim 1 to 6, wherein said rigidity imparting member (13) is disposed at said outer tube (2).

11. A catheter according to any of claim 1 to 6, wherein said rigidity imparting member (13) is disposed at both of said inner and outer tubes.

12. A catheter according to any of claim 1 to 6, wherein said outer tube (2) has said rigidity imparting member (13) extending in the axial direction and said rigidity imparting member is not disposed at the tip of said outer tube.

13. A catheter according to claim 12, wherein the portion not having said rigidity imparting member is formed with an annular member (25) disposed at the tip of said outer tube (2).

14. A catheter according to any of claim 1 to 6, wherein said inner tube (1) has said rigidity imparting member (13) extending in the axial direction from the proximal portion to the tip portion and, further, said rigidity imparting member is not disposed at the tip of said inner tube.

15. A catheter according to claim 14, wherein said rigidity imparting member disposed at said inner tube is provided at least from the proximal portion of said inner tube to the portion corresponding to the contractible or foldable portion of said expansible member (3).

16. A catheter according to claim 14, wherein the tip of said inner tube is formed with an annular member (25) attached to said tip of said inner tube and not having said rigidity imparting member.

17. A catheter according to any of claim 1 to 6, wherein said rigidity imparting member (13) is embedded inside of said inner tube or said outer tube.

18. A catheter according to any of claim 1 to 6, wherein said rigidity imparting member is embedded in the outer surface of said inner tube or said outer tube.

19. A catheter according to any of claim 1 to 6, wherein said rigidity imparting member is a braided member formed by wire material in a mesh-like manner.

20. A catheter according to claim 19, wherein said wire material is metal wire.

21. A catheter according to claim 19, wherein said wire material is made of synthetic fibers.

22. A catheter according to claim 21, wherein said synthetic fiber is any one of polyamide fibers, polyester fibers and polypropylene fibers.

23. A method of producing a catheter as set forth in claim 1, which comprises the steps of:
forming an inner tube (1) having a lumen opened from the tip to the rear end;
forming an outer tube (2) having a lumen opened from the tip to the rear end, with the inner diameter being larger than the outer diameter of said inner tube said outer tube being formed shorter by a predetermined length than said inner tube;
disposing a rigidity imparting member (13) at at least one of said inner and outer tubes,
forming separately a contractible or foldable expansible member (3) having a tip portion and a proximal portion;
inserting said inner tube to the inside of said outer tube;
securing said proximal portion of said expansible member to the tip portion of said outer tube; and
securing said tip portion of said expansible member to the tip portion of said inner tube.

24. A method according to claim 23, wherein said step of forming said inner tube (1) comprises forming a tube member and said step of disposing a rigidity imparting member (13) comprises disposing said rigidity imparting member at said tube member.

25. A method according to claim 23, wherein said step of forming said outer tube (2) comprises forming a tube member and said step of disposing a rigidity imparting member (13) comprises disposing said rigidity imparting member at said tube member.

26. A method according to claim 24 or 25, wherein said step of disposing said rigidity imparting member (13) comprises applying said rigidity imparting member to said tube member forming said inner tube (1) or said outer tube (2) and embedding the thus applied rigidity imparting member into said tube member.

27. A method according to any of claim 23 to 26, wherein said step of securing said tip portion of said expansible member (3) to said tip portion of said inner tube (1) is conducted after securing said proximal portion of said expansible member (3) to said tip portion of said outer tube (2) and then inserting said inner tube (1) into said outer tube (2) secured with said expansible member (3).

28. A method according to any of claim 23 to 27 wherein said step of securing said tip portion of said expansible member (3) to said tip portion of said inner tube (1) comprises securing said tip portion of said expansible member (3) by heat shrinkage under heating.

29. A method according to any of claim 23 to 28, wherein said step of securing said tip portion of said expansible member (3) to said tip portion of said inner tube (1) comprises securing said proximal portion of said expansible member by heat-shrinkage under heating.

30. A method according to any of claim 23 to 29, further comprising a step of forming an opening to the proximal portion of said inner tube (1) in communication with said lumen of said inner tube and a step of forming an opening to the proximal portion of said outer tube (2) in communication with said lumen of said outer tube.

## Patentansprüche

1. Katheter, versehen mit einem ausdehnbaren Element, das ein Innenrohr (1), das ein erstes Lumen (4) aufweist, dessen Spitze offen ist, ein Außenrohr (2), das koaxial mit dem Innenrohr angeordnet ist, wobei es ein zweites Lumen (6) zwischen ihm und der Außenfläche des Innenrohrs (1) bildet, ein zusammenziehbares oder zusammenlegbares ausdehnbares Element (3) aufweist, dessen Endabschnitt am Innenrohr (1) angebracht ist und dessen proximaler Abschnitt am Außenrohr (2) angebracht ist, und mit dem zweiten Lumen nahe dem proximalem Abschnitt in Verbindung steht, wobei eine mit dem ersten Lumen (4) in Verbindung stehende erste Öffnung (9) am proximalen Abschnitt des Innenrohrs (1) angeordnet ist, wobei eine mit dem zweiten Lumen (6) in Verbindung stehende zweite Öffnung (11) am proximalen Abschnitt des Außenrohrs (2) angeordnet ist, wobei sich das Ende des Außenrohrs (2) an einer Stelle befindet, die um eine vorbestimmte Länge vom Ende des Innenrohrs (1) mit Aussparung versehen ist, dadurch **gekennzeichnet,** daß ein Steifigkeit vermittelndes Element (13) bei mindestens einem von dem Innen- und Außenrohr so angeordnet ist, daß es sich in der axialen Richtung erstreckt.

2. Katheter nach Anspruch 1, bei dem das ausdehnbare Element (3) aus Polyolefin, Polyvinylchlorid, Polyamidelastomer oder Polyester hergestellt ist.

3. Katheter nach Anspruch 1 oder 2, bei dem das ausdehnbare Element (3) einen im wesentlichen zylindrischen Abschnitt (3a) aufweist, der einen ungefähr gleichen Durchmesser aufweist.

4. Katheter nach einem beliebigen Anspruch 1 bis 3, bei dem ein vorderer und ein hinterer Abschnitt des zylindrischen Abschnitts (3a) des ausdehnbaren Elements verjüngt sind.

5. Katheter nach Anspruch 2, bei dem das Polyolefin Polyethylen, Polypropylen, Ethylen-Vinylacetat-Copolymer oder vernetztes Ethylen-Vinylacetat-Copolymer ist.

6. Katheter nach einem beliebigen vorhergehenden Anspruch, bei dem die erste Öffnung und die zweite Öffnung bei einem verzweigten Buchsenteil (20) angeordnet sind, der an den proximalen Enden des Innenrohrs (1) und des Außenrohrs (2) angebracht ist.

7. Katheter nach beliebigen Ansprüchen 1 bis 6, bei dem eine Außenfläche des Außenrohrs und des ausdehnbaren Elements einer hydrophilen Behandlung unterzogen wurde, um Schmierung aufzuweisen, wenn die Außenfläche während der Benutzung mit Blut in Kontakt gebracht wird.

8. Katheter nach Anspruch 7, bei dem die hydrophile Behandlung durch Überziehen mit einem hydrophilen Polymer ausgeführt wird, das aus Poly(2-Hydroxyethylmethacrylat), Polyhydroxyethylacrylat, Hydroxypropylcellulose, Methylvinylether-Maleinsäureanhydrid-Copolymer, Polyethylenglykol, Polyacrylamid und Polyvinylpyrrolidon ausgewählt worden ist.

9. Katheter nach einem beliebigen vorhergehenden Anspruch, bei dem das Steifigkeit vermittelnde Element (13) am Innenrohr (1) angeordnet ist.

10. Katheter nach einem beliebigen von Anspruch 1 bis 6, bei dem das Steifigkeit vermittelnde Element (13) am Außenrohr (2) angeordnet ist.

11. Katheter nach einem beliebigen von Anspruch 1 bis 6, bei dem das Steifigkeit vermittelnde Element (13) am Innen- und am Außenrohr angeordnet ist.

12. Katheter nach einem beliebigen von Anspruch 1 bis 6, bei dem das Außenrohr (2) das Steifigkeit vermittelnde Element (13) aufweist, das sich in der axialen Richtung erstreckt, und das Steifigkeit vermittelnde Element nicht am Ende des Außenrohrs angeordnet ist.

13. Katheter nach Anspruch 12, bei dem der Abschnitt, der das Steifigkeit vermittelnde Element nicht aufweist, mit einem ringförmigen Element (25) gebildet ist, das am Ende des Außenrohrs (2) angeordnet ist.

14. Katheter nach einem beliebigen von Anspruch 1 bis 6, bei dem das Innenrohr (1) das Steifigkeit vermittelnde Element (13) aufweist, das sich in der axialen Richtung vom proximalen Abschnitt zum Endabschnitt erstreckt, und des weiteren das Steifigkeit vermittelnde Element nicht am Ende des Innenrohrs angeordnet ist.

15. Katheter nach Anspruch 14, bei dem das am Innenrohr angeordnete, Steifigkeit vermittelnde Element mindestens vom proximalen Abschnitt des Innenrohrs bis zu dem Abschnitt vorgesehen ist, der dem zusammenziehbaren oder zusammenlegbaren Abschnitt des ausdehnbaren Elements (3) entspricht.

16. Katheter nach Anspruch 14, bei dem das Ende des Innenrohrs mit einem ringförmigen Element (25) gebildet ist, das am Ende des Innenrohrs angebracht ist und nicht das Steifigkeit vermittelnde Element aufweist.

17. Katheter nach einem beliebigen von Anspruch 1 bis 6, bei dem das Steifigkeit vermittelnde Element (13) im Inneren des Innenrohrs oder des Außenrohrs eingebettet ist.

18. Katheter nach einem beliebigen von Anspruch 1 bis 6, bei dem das Steifigkeit vermittelnde Element in der Außenfläche des Innenrohrs oder des Außenrohrs eingebettet ist.

19. Katheter nach einem beliebigen von Anspruch 1 bis 6, bei dem das Steifigkeit vermittelnde Element ein geflochtenes Element ist, das auf maschenartige Weise durch Drahtmaterial gebildet ist.

20. Katheter nach Anspruch 19, bei dem das Drahtmaterial Metalldraht ist.

21. Katheter nach Anspruch 19, bei dem das Drahtmaterial aus synthetischen Fasern hergestellt ist.

22. Katheter nach Anspruch 21, bei dem die synthetische Faser irgendeine von Polyamidfasern, Polyesterfasern und Polypropylenfasern ist.

23. Verfahren, zur Herstellung eines Katheters nach Anspruch 1, das die Schritte umfaßt, daß:
- ein Innenrohr (1) geformt wird, das ein vom Vorderende bis zum hinteren Ende geöffnetes Lumen aufweist;
- ein Außenrohr (2) geformt wird, das ein vom Vorderende bis zum hinteren Ende geöffnetes Lumen aufweist, wobei der Innendurchmesser größer als der Außendurchmesser des Innenrohrs ist, wobei das Außenrohr um eine vorbestimmte Länge kürzer als das Innenrohr geformt ist;
- ein Steifigkeit vermittelndes Element (13) bei mindestens einem von dem Innen- und Außenrohr vorgesehen wird;
- ein zusammenziehbares oder zusammenlegbares ausdehnbares Element (3) getrennt geformt wird, das einen Endabschnitt und einen proximalen Abschnitt aufweist;
- das Innenrohr in das Innere des Außenrohrs eingesetzt wird;
- der proximale Abschnitt des ausdehnbaren Elements am Endabschnitt des Außenrohrs befestigt wird; und
- der Endabschnitt des ausdehnbaren Elements am Endabschnitt des Innenrohrs befestigt wird.

24. Verfahren nach Anspruch 23, bei dem der Schritt des Formens des Innenrohrs (1) das Formen eines Rohrelements umfaßt und der Schritt des Vorsehens eines Steifigkeit vermittelnden Elements (13) das Vorsehen des Steifigkeit vermittelnden Elements bei dem Rohrelement umfaßt.

25. Verfahren nach Anspruch 23, bei dem der Schritt des Formens des Außenrohrs (2) das Formen eines Rohrelements umfaßt und der Schritt des Vorsehens eines Steifigkeit vermittelnden Elements (13) das Vorsehen des Steifigkeit vermittelnden Elements bei dem Rohrelement umfaßt.

26. Verfahren nach Anspruch 24 oder 25, bei dem der Schritt des Vorsehens des Steifigkeit vermittelnden Elements (13) das Aufbringen des Steifigkeit vermittelnden Elements an dem das Innenrohr (1) oder das Außenrohr (2) bildenden Rohrelement und das Einbetten des so aufgebrachten, Steifigkeit vermittelnden Elements in das Rohrelement umfaßt.

27. Verfahren nach einem beliebigen von Anspruch 23 bis 26, bei dem der Schritt des Befestigens des Endabschnitts des ausdehnbaren Elements (3) am Endabschnitt des Innenrohrs (1) ausgeführt wird, nachdem der proximale Abschnitt des ausdehnbaren Elements (3) am Endabschnitt des Außenrohrs (2) befestigt ist und dann das Innenrohr (1) in das Außenrohr (2) eingeführt ist, befestigt mit dem ausdehnbaren Element (3).

28. Verfahren nach einem beliebigen von Anspruch 23 bis 27, bei dem der Schritt der Befestigung des Endabschnitts des ausdehnbaren Elements (3) am Endabschnitt des Innenrohrs (1) umfaßt, daß der Endabschnitt des ausdehnbaren Elements (3) durch Wärmeschrumpfen bei Erwärmung befestigt wird.

29. Verfahren nach einem beliebigen von Anspruch 23 bis 28, bei dem der Schritt der Befestigung des Endabschnitts des ausdehnbaren Elements (3) am Endabschnitt des Innenrohrs (1) umfaßt, daß der proximale Abschnitt des ausdehnbaren Elements durch Wärmeschrumpfen bei Erwärmung befestigt wird.

30. Verfahren nach einem beliebigen von Anspruch 23 bis 29, weiterhin umfassend einen Schritt, daß eine Öffnung am proximalen Abschnitt des Innenrohrs (1) in Verbindung mit dem Lumen des Innenrohrs gebildet wird, und einen Schritt, daß eine Öffnung am proximalen Abschnitt des Außenrohrs (2), in Verbindung mit dem Lumen des Außenrohrs gebildet wird.

## Revendications

1. Cathéter muni d'un élément dilatable, qui comprend un tube intérieur (1) avec une première lumière (4) dont le bout est ouvert, un tube extérieur (2) placé coaxialement avec ledit tube intérieur en formant une seconde lumière (6) entre lui et la surface extérieure dudit tube intérieur (1), un élément dilatable (3) pouvant se contracter ou se replier avec une partie d'extrémité fixée audit tube intérieur (1) et une partie proximale fixée audit tube extérieur (2), et communiquant avec ladite seconde lumière près de ladite partie proximale, un premier orifice (9) qui communique avec ladite première lumière (4) placé au niveau de la partie proximale dudit tube intérieur (1), un second orifice (11) qui communique avec ladite seconde lumière (6) placé au niveau de la partie proximale dudit tube extérieur (2), le bout dudit tube extérieur (2) étant en une position rétractée d'une longueur prédéterminée par rapport au bout dudit tube intérieur (1), caractérisé en ce qu'un élément de rigidification (13) est placé au niveau de l'un au moins desdits tubes intérieur et extérieur pour s'étendre dans la direction axiale.

2. Cathéter selon la revendication 1, dans lequel ledit élément dilatable (3) est fait de polyoléfine, de poly(chlorure de vinyle), d'un élastomère polyamide ou de polyester.

3. Cathéter selon la revendication 1 ou 2, dans lequel ledit élément dilatable (3) a une partie (3a) sensiblement cylindrique ayant un diamètre approximativement égal.

4. Cathéter selon l'une quelconque des revendications 1 à 3, dans lequel une partie avant et une partie arrière de ladite partie cylindrique (3a) dudit élément dilatable sont coniques.

5. Cathéter selon la revendication 2, dans lequel ladite polyoléfine est du polyéthylène, du polypropylène, un copolymère éthylène/acétate de vinyle ou un copolymère réticulé éthylène/acétate de vinyle.

6. Cathéter selon l'une quelconque des précédentes revendications, dans lequel ledit premier orifice et ledit second orifice sont placés au niveau d'un manchon bifurqué (20) fixé aux extrémités proximales dudit tube intérieur (1) et dudit tube extérieur (2).

7. Cathéter selon l'une quelconque des revendications 1 à 6, dans lequel une surface extérieure dudit tube extérieur et dudit élément dilatable a été soumise à un traitement hydrophile de façon à présenter une caractéristique de lubrification quand ladite surface extérieure est amenée en contact avec le sang pendant l'utilisation.

8. Cathéter selon la revendication 7, dans lequel ledit traitement hydrophile est effectué par dépôt d'un polymère hydrophile choisit parmi le poly(méthacrylate de 2-hydroxyéthyle), le polyhydroxyéthylacrylate, l'hydroxypropylcellulose, un copolymère d'oxyde de méthyle et de vinyle et d'anhydride maléique, le polyéthylèneglycol, le polyacrylamide et le polyvinlypyrrolidone.

9. Cathéter selon l'une quelconque des précédentes revendications, dans lequel ledit élément de rigidification (13) est placé au niveau dudit tube intérieur (1).

10. Cathéter selon l'une quelconque des revendications 1 à 6, dans lequel ledit élément de rigidification (13) est placé au niveau dudit tube extérieur (2).

11. Cathéter selon l'une quelconque des revendications 1 à 6, dans lequel ledit élément de rigidification (13) est placé au niveau des deux tubes intérieur et extérieur.

12. Cathéter selon l'une quelconque des revendications 1 à 6, dans lequel ledit tube extérieur (2) a ledit élément de rigidification (13) qui s'étend dans la direction axiale et ledit élément de rigidification n'est pas placé au niveau du bout dudit tube extérieur.

13. Cathéter selon la revendication 12, dans lequel la partie n'ayant pas ledit élément de rigidification est formée avec un élément annulaire (25) placé au niveau du bout dudit tube extérieur (2).

14. Cathéter selon l'une quelconque des revendications 1 à 6, dans lequel ledit tube intérieur (1) a ledit élément de rigidification (13) qui s'étend dans la direction axiale depuis la partie proximale jusqu'à la partie d'extrémité et, en outre, ledit élément de rigidification n'est pas placé au niveau du bout dudit tube intérieur.

15. Cathéter selon la revendication 14, dans lequel ledit élément de rigidification placé au niveau dudit tube intérieur est placé au moins de la partie proximale dudit tube intérieur jusqu'à la partie correspondant à la partie qui peut se contracter ou se replier dudit élément dilatable (3).

16. Cathéter selon la revendication 14, dans lequel le bout dudit tube intérieur est formé avec un élément annulaire (25) fixé audit bout dudit tube intérieur et n'ayant pas ledit élément de rigidification.

17. Cathéter selon l'une quelconque des revendications 1 à 6, dans lequel ledit élément de rigidification (13) est noyé à l'intérieur dudit tube intérieur ou du tube extérieur.

18. Cathéter selon l'une quelconque des revendications 1 à 6, dans lequel ledit élément de rigidification est noyé dans la surface extérieure dudit tube intérieur ou dudit tube extérieur.

19. Cathéter selon l'une quelconque des revendications 1 à 6, dans lequel ledit élément de rigidification est un élément tressé formé par un matériau en fil à la façon d'un treillis.

20. Cathéter selon la revendication 19, dans lequel ledit matériau en fil est un fil métallique.

21. Cathéter selon la revendication 19, dans lequel ledit matériau en fil est fait de fibres synthétiques.

22. Cathéter selon la revendication 21, dans lequel ladite fibre synthétique est l'une quelconque de fibres polyamide, de fibres polyester et de fibres polypropylène.

23. Procédé de fabrication d'un cathéter tel que revendiqué à la revendication 1, qui comprend les étapes consistant à :
- former un tube intérieur (1) ayant une lumière ouverte depuis le bout jusqu'à l'extrémité arrière,
- former un tube extérieur (2) ayant une lumière ouverte depuis le bout jusqu'à l'extrémité arrière, le diamètre intérieur étant plus grand que le diamètre extérieur dudit tube intérieur, ledit tube extérieur étant plus court d'une longueur prédéterminée que ledit tube intérieur,
- placer un élément de rigidification (13) au niveau de l'un au moins desdits tubes intérieur et extérieur,
- former séparément un élément dilatable (3) qui peut se contracter ou se replier, avec une partie d'extrémité et une partie proximale,
- insérer ledit tube intérieur à l'intérieur dudit tube extérieur,
- fixer ladite partie proximale dudit élément dilatable à la partie de bout dudit tube extérieur, et
- fixer ladite partie d'extrémité dudit élément dilatable à la partie de bout dudit tube intérieur.

24. Procédé selon la revendication 23, dans lequel ladite étape de formation dudit tube intérieur (1) comprend la formation d'un élément de tube et ladite étape de mise en place d'un élément de rigidification (13) comprend la mise en place dudit élément de rigidification au niveau dudit élément de tube.

25. Procédé selon la revendication 23, dans lequel ladite étape de formation dudit tube extérieur (2) comprend la formation d'un élément de tube et ladite étape de mise en place d'un élément de rigidification (13) comprend la mise en place dudit élément de rigidification au niveau dudit élément de tube.

26. Procédé selon la revendication 24 ou 25, dans lequel ladite étape de mise en place dudit élément de rigidification (13) comprend l'application dudit élément de rigidification sur ledit élément de tube formant ledit tube intérieur (1) ou ledit tube extérieur (2) et le noyage de l'élément de rigidification ainsi mis en place dans ledit élément de tube.

27. Procédé selon l'une quelconque des revendications 23 à 26, dans lequel ladite étape de fixation de ladite partie d'extrémité dudit élément dilatable (3) à ladite partie de bout dudit tube intérieur (1) est effectuée après fixation de ladite partie proximale dudit élément dilatable (3) à ladite partie de bout dudit tube extérieur (2), suivie de l'insertion dudit tube intérieur (1) dans ledit tube extérieur (2) fixé audit élément dilatable (3).

28. Procédé selon l'une quelconque des revendications 23 à 27, dans lequel ladite étape de fixation de ladite partie d'extrémité dudit élément dilatable (3) à ladite partie de bout dudit tube intérieur (1) comprend la fixation de ladite partie d'extrémité de l'élément dilatable (3) par rétrécissement à la chaleur.

29. Procédé selon l'une quelconque des revendications 23 à 28, dans lequel ladite étape de fixation de ladite partie d'extrémité dudit élément dilatable (3) à ladite partie de bout dudit tube intérieur (1) comprend la fixation de ladite partie proximale dudit élément dilatable par rétrécissement à la chaleur.

30. Procédé selon l'une quelconque des revendications 23 à 29, comprenant en outre une étape qui consiste à former un orifice dans la partie proximale dudit tube intérieur (1), en communication avec ladite lumière dudit tube intérieur, et une étape qui consiste à former un orifice dans la partie proximale dudit tube extérieur (2), en communication avec ladite lumière dudit tube extérieur.
